(19) Europäisches Patentamt / European Patent Office / Office européen des brevets

(11) **EP 4 194 875 B1**

(12) # EUROPÄISCHE PATENTSCHRIFT

(45) Veröffentlichungstag und Bekanntmachung des Hinweises auf die Patenterteilung:
**22.05.2024   Patentblatt 2024/21**

(21) Anmeldenummer: **23155132.6**

(22) Anmeldetag: **02.10.2018**

(51) Internationale Patentklassifikation (IPC):
**G01R 33/36** *(2006.01)*      **G01R 33/565** *(2006.01)*
**G01R 33/422** *(2006.01)*

(52) Gemeinsame Patentklassifikation (CPC):
**G01R 33/36; G01R 33/422; G01R 33/5659**

(54) **MAGNETRESONANZTOMOGRAPH MIT AKTIVER STÖRUNTERDRÜCKUNG UND VERFAHREN ZUR STÖRUNTERDRÜCKUNG IN EINEM MAGNETRESONANZTOMOGRAPHEN**

MAGNETIC RESONANCE TOMOGRAPH WITH ACTIVE INTERFERENCE SUPPRESSION AND METHOD FOR SUPPRESSING INTERFERENCE IN A MAGNETIC RESONANCE TOMOGRAPH

TOMOGRAPHE À RÉSONANCE MAGNÉTIQUE À SUPPRESSION ACTIVE DES PARASITES ET PROCÉDÉ D'ÉLIMINATION DES PARASITES DANS UN TOMOGRAPHE À RÉSONANCE MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.10.2017   EP 17194969**

(43) Veröffentlichungstag der Anmeldung:
**14.06.2023   Patentblatt 2023/24**

(62) Dokumentnummer(n) der früheren Anmeldung(en) nach Art. 76 EPÜ:
**18785882.4 / 3 658 946**

(73) Patentinhaber: **Siemens Healthineers AG**
**91301 Forchheim (DE)**

(72) Erfinder:
• **Biber, Stephan**
  **91056 Erlangen (DE)**
• **Grodzki, David**
  **91058 Erlangen (DE)**
• **Vester, Markus**
  **90471 Nürnberg (DE)**
• **Nichols, Ian Edward**
  **Fair Oak, Eastleigh, Hampshire, SO50 7HA (GB)**
• **Sadler, David James**
  **Eastleigh, Hamphire, SO53 3BS (GB)**

(74) Vertreter: **Siemens Healthineers**
**Patent Attorneys**
**Siemens Healthcare GmbH**
**SHS TE IP**
**Henkestraße 127**
**91052 Erlangen (DE)**

(56) Entgegenhaltungen:
**WO-A1-2015/150236      DE-A1-102012 203 450**
**US-A1- 2008 048 658**

• **KUZMIN V V ET AL: "An improved shielded RF transmit coil for low-frequency NMR and MRI", JOURNAL OF MAGNETIC RESONANCE, ACADEMIC PRESS, ORLANDO, FL, US, Bd. 256, 14. Mai 2015 (2015-05-14), Seiten 70-76, XP029224704, ISSN: 1090-7807, DOI: 10.1016/J.JMR.2015.05.001**
• **BURL M ET AL: "EXAMPLES OF THE DESIGN OF SCREENED AND SHIELDED RF RECEIVER COILS", MAGNETIC RESONANCE IN MEDICINE, WILEY-LISS, US, Bd. 36, Nr. 2, 1. August 1996 (1996-08-01) , Seiten 326-330, XP000625808, ISSN: 0740-3194**

**Beschreibung**

Die Erfindung betrifft ein Verfahren zur aktiven Störunterdrückung in einem Magnetresonanztomographen sowie einen Magnetresonanztomographen mit einem Empfänger.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden. Die Empfangsantennen können auch in einer Patientenliege verbaut sein.

Magnetresonanztomographen erfordern in zweierlei Hinsicht eine Hochfrequenzabschirmung. Zum einen werden zur Anregung der Kernspins Hochfrequenzimpulse mit Leistungen im Kilowattbereich erzeugt, die nur teilweise im Patienten absorbiert werden. Radiowellen, die die Patientendurchführung verlassen, werden in den Raum abgestrahlt und müssen daher zur Einhaltung von Emissionsgrenzwerten abgeschirmt werden.

Umgekehrt sind die für die Bildgebung zu empfangenden Magnetresonanzsignale extrem schwach. Um hier ein ausreichendes Signal-zu-Rausch-Verhältnis (SNR) zu erreichen, ist eine Abschirmung externer Störsignal erforderlich.

Deshalb werden im Stand der Technik um einen Magnetresonanztomographen aufwändige Schirmkabinen installiert, um sowohl Emissionen als auch Immissionen zu reduzieren.

Aus der Druckschrift DE 10 2014 207 843 ist eine Kniespule mit einer lokalen Schirmung bekannt.

Aus dem Dokument WO2015/150236 ist ein Verfahren und ein Magnetresonanztomograph bekannt, wobei der Magnetresonanztomograph Magnetresonanzdaten mit aktivierter Hochfrequenzanregung erfasst. Hochfrequenz-Rauschsignale werden mit zumindest einer Rauschsignalempfangsspule erfasst, wobei das Rauschsignal mit den bildgebenden Magnetresonanzdaten erfasst wird. Kalibrierungs-Magnetresonanzsignale werden mit deaktivierter Hochfrequenzanregung erfasst. Referenz-Hochfrequenzdaten werden mit der Rauschsignalempfangsspule erfasst, wobei die Referenz-Hochfrequenzdaten gleichzeitig mit den erfasst werden. Eine Rauschkalibrierung wird unter Verwendung der Referenz-Hochfrequenzdaten und der Kalibrierungs-Magnetresonanzsignale ermittelt.

Das Dokument US 2008/048658 A1 offenbart eine Signalverarbeitungseinrichtung für eine aktive Störunterdrückung von elektronischen Störsignalen aus einer Umgebung. Die Vorrichtung verwendet eine Rauscherfassungsspule außerhalb des Bilderfassungsbereichs um ein Rauschsignal in der Umgebung zu erfassen und zieht das korrelierte Rauschen in dem Umgebungssignal von dem Bildsignal ab.

Aus dem Dokument KUZMIN V V ET AL: "An improved shielded RF transmit coil for low-frequency NMR and MRI", JOURNAL OF MAGNETIC RESONANCE, ACADEMIC PRESS, ORLANDO, FL, US, Bd. 256, 14. Mai 2015 (2015-05-14), Seiten 70-76, XP029224704, ISSN: 1090-7807, DOI: 10.1016/J.JMR.2015.05.001 ist eine aktiv abgeschirmte zylindrische Hochfrequenz-Sendespule bekannt, die eine höchst homogene interne Feldverteilung über ein erweitertes Volumen und ein stark unterdrücktes externes Feld aufweist.

Das Dokument BURL MET AL: "EXAMPLES OF THE DESIGN OF SCREENED AND SHIELDED RF RECEIVER COILS", MAGNETIC RESONANCE IN MEDICINE, WILEY-LISS, US, Bd. 36, Nr. 2, 1. August 1996 (1996-08-01), Seiten 326-330, XP000625808, ISSN: 0740-3194 beschreibt drei unterschiedliche Arten zur Abschirmung von Hochfrequenz-Oberflächenspulen auf. Es ist das Ziel, eine Oberflächenspule bereitzustellen, die eine wesentlich höhere Empfindlichkeit auf einer Seite als auf einer anderen Seite aufweist, sodass ein Signal- und Rauschanteil aus unerwünschten Bereichen minimiert werden kann.

Aus dem Dokument DE 10 2012 203450 A1 ist eine Magnetresonanzeinrichtung mit einer zylindrischen Patientenaufnahme bekannt. Die Magnetresonanzeinrichtung umfasst einen die Patientenaufnahme umgebend angeordneten Hochfrequenzschirm zur Abschirmung von wenigstens einer Hochfrequenzspule gegen eine Gradientenspulenanordnung auf. Der Hochfrequenzschirm ist zur Bildung eines Abschirmraums erweitert, der die Hochfrequenzspule gegen Hochfrequenzsignale außerhalb des Abschirmraums abschirmt.

Es könnte daher eine Aufgabe der Erfindung sein, den Aufwand für eine Abschirmung zu reduzieren.

Die Aufgabe wird durch einen erfindungsgemäßen Magnetresonanztomographen nach Anspruch 1 und Anspruch 2 sowie durch ein erfindungsgemäßes Verfahren zum Betrieb des Magnetresonanztomographen nach Anspruch 6 und Anspruch 7 gelöst.

Der erfindungsgemäße Magnetresonanztomograph weist in der in Anspruch 1 definierten erfindungsgemäßen Variante einen Patiententunnel, eine erste Empfangsantenne zum Empfang eines Magnetresonanzsignals aus einem Patienten, wobei der Patient in dem Patiententunnel ist, eine zweite Empfangsantenne zum Empfang eines Signals mit der Larmorfrequenz des Magnetresonanzsignals und einen Empfänger auf. Der Empfänger steht mit der ersten Empfangsantenne und der zweiten Empfangsantenne in Signalverbindung und ist vorzugsweise ausgelegt, das Magnetresonanzsignal für eine Bildgebung aufzubereiten.

Die zweite Empfangsantenne ist außerhalb oder in der Nähe einer Öffnung des Patiententunnels angeordnet. Als Öffnung des Patiententunnels werden insbesondere die Öffnungen angesehen, durch die die Patientenliege mit dem Patienten in den Patiententunnel gefahren werden und, je nach zu untersuchendem Bereich, am gegenüberliegenden Ende den Patiententunnel auch wieder verlässt. In der Nähe wird dabei ein Abstand von der Öffnung von weniger als 0,1 m, 0,2 m, 0,5 m, 1 m oder 2 m angesehen. In der Nähe kann auch als ein Abstand von der Öffnung von weniger als einer viertel Wellenlänge oder einer halben Wellenlänge einer Radiowelle in der Luft mit einer Larmorfrequenz des Magnetresonanztomographen angesehen werden.

Die erste Empfangsantenne empfängt dabei bevorzugt das Magnetresonanzsignal, das aber immer auch einen kleinen Anteil des Störsignals aufweist. Umgekehrt empfängt die zweite Empfangsantenne nicht nur das Störsignal, sondern auch einen minimalen bzw. vernachlässigbaren Anteil des Magnetresonanzsignals. Der Einfachheit halber wird dennoch im Folgenden das von der ersten Empfangsantenne empfangene Signal als Magnetresonanzsignal (wenn auch mit einem zu beseitigenden Anteil des störenden Signals) und das von der bzw. den zweiten Empfangsantennen empfangene Signal als Störsignal bezeichnet.

Der Empfänger ist dabei ausgelegt, ein mit der zweiten Empfangsantenne empfangenes Störsignal in einem von der ersten Empfangsantenne empfangenen Magnetresonanzsignal zu unterdrücken. Beispielhafte Ausführungsformen sind zu den Unteransprüchen näher beschrieben.

In der erindungsgemäßen Variante des Anspruchs 2 wird dabei insbesondere die Möglichkeit betrachtet, dass das Störsignal breitbandiger ist als das Magnetresonanzsignal. Die Anteile außerhalb des Frequenzbereichs des Magnetresonanzsignals korrelieren dabei mit den Anteilen innerhalb des Frequenzbereichs des Magnetresonanzsignals. Es kann daher gemäß der Erfindung auch hinreichend sein, wenn die zweite Empfangsantenne das breitbandige Störsignal empfängt und der Empfänger ausgelegt ist, dieses Signal nur teilweise, beispielsweise in einem Frequenzbereich ungleich bzw. außerhalb des Frequenzbereichs des Magnetresonanzsignals auszuwerten und dann in Abhängigkeit von diesem Teilsignal das Störsignal in einem von der ersten Empfangsantenne empfangenen Magnetresonanzsignal zu unterdrücken. Denkbar wäre es aber auch alternativ oder ergänzend, dass bereits die zweite Empfangsantenne nur Frequenzen außerhalb des Frequenzbereichs des Magnetresonanzsignals empfängt und diese an den Empfänger weiterleitet. Aufgrund der Korrelation kann beispielsweise die Amplitude der Frequenzanteile des Störsignals im Frequenzbereich des Magnetresonanzsignals mit einer Amplitude außerhalb verknüpft sein. Auf diese Weise ist es auch denkbar, dass die zweite Empfangsantenne in Verbindung mit dem Empfänger während einer Magnetresonanzmessung nur Frequenzanteile außerhalb des Frequenzbereichs des Magnetresonanzsignals auf Störsignale überwacht und in Abhängigkeit davon das Störsignal in den Signalen der ersten Empfangsantenne unterdrückt. Möglich wäre es dabei beispielsweise, dass der Empfänger einen Zusammenhang zwischen Störsignal aus der ersten Empfangsantenne und Störsignalanteil aus der zweiten Empfangsantenne, auch als Transferfunktion bezeichnet, für ein Störsignal im Frequenzbereich der Magnetresonanzsignale zwischen Erfassungen von Magnetresonanzsignalen ermittelt und während der Erfassung von Magnetresonanzsignalen beispielsweise die Amplitude bzw. Skalierung anhand der von der zweiten Empfangsantenne außerhalb des Frequenzbereichs der Magnetresonanzsignale empfangenen Anteile des Störsignals anpasst.

Denkbar ist es auch, dass zwischen der zweiten Empfangsantenne und dem Empfänger ein Filter vorgesehen ist, dass bevorzugt das Störsignal durchlässt und Magnetresonanzsignale unterdrückt. Beispielsweise könnte für einen Störer mit Anteilen außerhalb des Frequenzbandes des Magnetresonanzsignals ein Filter vorgesehen sein, das die Magnetresonanzsignale unterdrückt. Das Filter könnte auch adaptiv oder steuerbar sein. So wäre es denkbar, dass die Entstörsteuerung das Filter an die Larmorfrequenz einer aktuell erfassten Schicht anpasst

Nachfolgend ist erläutert, wie die Unterdrückung des Störsignals in den Signalen der zweiten Empfangsantenne durch den Empfänger erfolgen kann. Beispielsweise kann der Empfänger eine Summationseinrichtung aufweisen, die eine von einem oder mehreren Parametern abhängige Linearkombination von Magnetresonanzsignal und Störsignal bildet. Weiterhin kann der Empfänger eine Entstörsteuerung aufweisen, die ausgelegt ist, den oder die Parameter so zu variieren, dass eine Energie des Störsignals in der Linearkombination minimal wird. Dabei können einer oder mehrere Parameter komplex sein, um eine Phasenverschiebung zu modellieren oder eine Phasenverschiebung durch einen eigenen Parameter angegeben sein. Mehrere Parameter erlauben insbesondere die effektive Unterdrückung unterschiedlicher Störquellen.

Denkbar sind aber auch nicht-lineare Kombinationen der Signale in Abhängigkeit von dem bzw. den Parametern Die Entstörsteuerung könnte auch Störsignale mit besonders großer Amplitude gegenüber schwächeren Störsignalen bei der Störunterdrückung in den Parametern besonders stark gewichten, denn durch einen besonders großen Abstand des Signalpegels gegenüber einem statistischen Hintergrundrauschen lassen sich diese starken Störsignale besonders gut unterdrücken. Als unterschiedliche Störsignale werden dabei insbesondere Störsignale angesehen, die sich durch unterschiedlichen Ursprungsort mittels mehrerer zweiten Empfangsantennen trennen lassen, unterschiedliche Frequenzbereiche belegen oder sich durch ein unterschiedliches zeitliches Verhalten unterscheiden.

Der Empfänger kann dabei ausgelegt sein, diese erfindungsgemäße Verarbeitung des empfangenen Magnetresonanzsignals und des Störsignals zur Unterdrückung des Störsignals in Echtzeit auszuführen, beispielsweise mittels einer programmierbaren Logik (FPGA) oder eines Signalprozessors (DSP).

Es ist aber auch möglich, dass der Empfänger einen Speicher aufweist und das empfangene Störsignal und das empfangene Magnetresonanzsignal zunächst speichert, wobei die Unterdrückung des Störsignals erst zu einem späteren Zeitpunkt mit einer Verzögerung beispielsweise von der Dauer einer Echosequenz, einer Anregungssequenz oder einer ganzen Bilderfassung einer einzelnen Schicht oder der ganzen Bilderfassungssequenz erfolgt. Die Verzögerung kann beispielsweise größer als 50 ms, 100 ms, 0,5 s, 1 s, 10s 1 min oder mehr sein.

Als Empfänger im Sinne der Erfindung kann dabei die Hardware zur analogen und/oder digitalen Hochfrequenzverarbeitung wie beispielsweise Verstärker, Filter und Mischer in Echtzeit, aber auch eine Bildauswerteeinheit zur späteren Erzeugung einer Abbildung aus den empfangenen Magnetresonanzsignalen angesehen werden.

Das erfindungsgemäße Verfahren zum Betrieb des erfindungsgemäßen Magnetresonanztomographen weist den Schritt auf, mittels des Empfängers ein Störsignal über die zweite Empfangsantenne zu empfangen. Aufgrund der Anordnung der zweiten Antenne in der Nähe der Öffnung weist das von der zweiten Antenne empfangene Störsignal kaum oder gar keine Anteile des Magnetresonanzsignals auf.

In einem anderen Schritt empfängt der Empfänger ein Magnetresonanzsignal über die erste Empfangsantenne. Bei der ersten Empfangsantenne kann es sich beispielsweise um eine Körperspule oder eine Lokalspule des Magnetresonanztomographen handeln.

In einem weiteren Schritt des Verfahrens verarbeitet der Empfänger das Magnetresonanzsignal in Abhängigkeit von dem Störsignal durch den Empfänger zu einem Empfangssignal, wobei die Abhängigkeit von einem Parameter abhängt. Beispielsweise ist es denkbar, dass der Empfänger eine Linearkombination aus Störsignal und Magnetresonanzsignal mit dem Parameter als Faktor bildet, wobei der Parameter komplex sein kann, um eine Phasenverschiebung abzubilden. Auch sind mehrere Parameter denkbar.

In einer anderen Variante des erfindungsgemäßen Verfahrens ist es vorgesehen, dass der Empfänger mittels der zweiten Empfangsantenne Anteile eines breitbandigen Störsignals außerhalb des Frequenzbereichs des Magnetresonanzsignals empfängt. Dabei korrelieren Anteile des Störsignals außerhalb des Frequenzbereichs des Magnetresonanzsignals mit Anteilen innerhalb des Frequenzbereichs. Beispielsweise ist es denkbar, dass die Amplituden des Störsignals innerhalb und außerhalb des Frequenzbereichs des Magnetresonanzsignals proportional zueinander sind, wenn es sich um die gleiche Quelle handelt. In der nachfolgend beschriebenen Unterdrückung des Störsignals in den von der ersten Empfangsantenne empfangenen Magnetresonanzsignalen kann dann beispielsweise als Abhängigkeit die Skalierung mit der Amplitude des Signals der zweiten Empfangsantenne vorsehen.

Vorteilhafter Weise kann durch die getrennten Frequenzbereiche eine Beeinflussung der Unterdrückung des Störsignals durch die Magnetresonanzsignale während einer Bilderfassung vermieden werden. Mit anderen Worten, durch die Auswertung des von der zweiten Empfangsantenne empfangenen Signals durch den Empfänger in Frequenzbereichen ungleich den Frequenzen der Magnetresonanzsignale kann vermieden werden, dass eingestreute Magnetresonanzsignale als Störsignal bewertet werden und durch die Entstörsteuerung unterdrückt werden.

Vorzugsweise berücksichtigt dabei die Entstörsteuerung auch unterschiedliche Eigenschaften von Signalen im Frequenzband der Magnetresonanzsignale und außerhalb. Dies können beispielsweise unterschiedliche Dämpfungen oder Signalverzögerungen sein, die durch andere Verstärkungsfaktoren und Phasenverschiebungen berücksichtigt werden können. Die unterschiedlichen Eigenschaften können beispielsweise durch Kalibriermessungen zur Bestimmung von Transferfunktionen ermittelt werden, wie sie zu den Unteransprüchen nachfolgend erläutert werden.

Dabei ist es denkbar, dass Störsignale zwischen und/oder auch während MR-Signalerfassungen von dem Empfänger erfasst werden. Eine permanente Erfassung erlaubt es auf vorteilhafte Weise, den zeitlichen Verlauf eines Störsignals besser zu erfassen und damit auch für die Zukunft besser abzuschätzen, was eine genauere Unterdrückung erlaubt.

Eine zwischenzeitliche Erfassung, d.h. zwischen der Erfassung von MR-Signalen, ermöglicht es auf der anderen Seite, die Bewertung von MR-Signalen als Störsignalen zu vermeiden. Es ist dabei auch denkbar, dass nicht nur die zweite bzw. die zweiten Empfangsantennen dabei die Störsignale erfassen, sondern auch gleichzeitig die erste Empfangsantenne bzw. ersten Empfangsantennen, sodass genau bekannt ist, wie ein von den zweiten Empfangsantennen empfangenes Störsignal von den ersten Empfangsantennen erfasst wird. Dieser auch als Transferfunktion bezeichnete Zusammenhang erlaubt dann eine genauere Bestimmung von Parametern zur Störunterdrückung und damit eine bessere Unterdrückung der Störung durch den Empfänger.

Es können die unterschiedlichen Verfahren (unterschiedliche Frequenz, zeitliche Verschränkung) auch miteinander kombiniert und/oder alternierend eingesetzt werden, um insgesamt zu einer verbesserten Entstörung zu gelangen.

Weiterhin ist es auch denkbar, dass eine Mittelung der Ergebnisse über längere oder mehrere Erfassungszeiträume und/oder auch zwischen den unterschiedlichen Verfahren erfolgt.

In einem anderen Schritt stellt der Empfänger, beispielsweise mittels einer Entstörsteuerung, den Parameter so ein, dass ein Anteil des Störsignals in dem Empfangssignal reduziert wird. Denkbar ist es beispielsweise, dass der Parameter durch die Entstörsteuerung in einem Optimierungsverfahren so eingestellt wird, dass eine Energie des Störsignals in dem Empfangssignal minimiert wird.

Wie bereits zuvor zum Empfänger erläutert, kann der Empfänger dabei das Magnetresonanzsignal und/oder das Störsignal auch speichern, sodass die Schritte des Verarbeitens und des Einstellens des Parameters auch mit zeitlichem Abstand zum Empfangen der Signale durch die erste Empfangsantenne und die zweite Empfangsantenne erfolgen können.

Auf vorteilhafte Weise ermöglicht es der erfindungsgemäße Magnetresonanztomograph und das Verfahren zum Betrieb durch die zweite Empfangsantenne und den erfindungsgemäßen Empfänger, den Anteil externer Störsignale in dem Magnetresonanzsignal zu reduzieren und daher mit einfacheren und kostengünstigeren Abschirmmaßnahmen auszukommen.

Weitere vorteilhafte Ausführungsformen sind in den Unteransprüchen angegeben.

In einer möglichen Ausführungsform des erfindungsgemäßen Magnetresonanztomographen ist der Magnetresonanztomograph ausgelegt, Magnetresonanzsignale mit einer Larmorfrequenz in einem Industrieband zu empfangen. Als Larmorfrequenz des Magnetresonanztomographen wird dabei eine Resonanzfrequenz der für die Bildgebung in dem Magnetresonanztomographen genutzten Kernspins in einem statischen Magnetfeld B0 eines Feldmagneten des Magnetresonanztomographen bezeichnet. Als Industrieband werden für eine Nutzung durch medizinische oder technische Geräte freigegebene Frequenzbänder bezeichnet, für die erleichterte Vorschriften bei der Emission und Genehmigung vorliegen. Diese werden auch als ISM-Bänder (Industrial, Scientific, Medical Band) bezeichnet. Ein beispielhaftes Frequenzband, in dem auch mit großen Leistungen emittiert werden darf, liegt zwischen 26.9 und 27,3 MHz. Andere derartige Frequenzbänder liegen zwischen 6,7 MHz und 6,8 MHz, 13,5 MHz und 13,6 MHz, 40,6 MHz und 40,7 MHz sowie 433,0 MHz und 434,8 MHz.

Ein Magnetresonanztomograph ist nicht nur darauf angewiesen, dass möglichst geringe Störungen empfangen werden, sondern die starken Anregungsimpulse dürfen auch andere Geräte nicht stören. Im ISM-Band sind die gesetzlichen Toleranzgrenzen wesentlich höher, sodass eine gesetzeskonforme Begrenzung durch Schirmung der emittierten Anregungspulse bei dieser Frequenz einfacher möglich ist oder auch nicht erforderlich ist. In synergistischer Verbindung mit der aktiven Schirmung, um empfangsseitig Störungen durch andere Geräte im ISM-Band zu unterdrücken, kann so auf vorteilhafte Weise ein Magnetresonanztomograph ganz ohne Schirmkabine realisiert werden.

In einer möglichen Ausführungsform weist der Magnetresonanztomograph dabei einen Sendepfad zum Aussenden eines Anregungspulses mit einem Filter auf. Der Filter ist dabei ausgelegt, Signale außerhalb des ISM-Bandes zu unterdrücken. Beispielsweise kann es sich bei dem Filter um einen Bandpassfilter für das verwendete ISM-Band handeln, das Frequenzen außerhalb des ISM-Bandes um mehr als 12 dB, 24 DB, 40 dB oder 60 dB relativ zu einem Signal mit minimaler Dämpfung innerhalb des ISM-Bandes dämpft. Je nach Ausführung der Hochfrequenzerzeugung des Magnetresonanztomographen kann der Filter beispielsweise zwischen Endstufe und einem Hybridkoppler, zwischen Hybridkoppler und Sende/Empfangs-Weiche oder zwischen Sende/Empfangs-Weiche und Sendeantenne angeordnet werden.

Der Filter ermöglicht auf vorteilhafte Weise, gesendete Hochfrequenzleistung im Wesentlichen auf das ISM-Band zu beschränken und so die schärferen Grenzwerte außerhalb des ISM-Bandes einzuhalten. So kann auch ohne RF-Kabine der Magnetresonanztomograph im ISM-Band betrieben werden. Grundsätzlich ist es aber auch denkbar, ein ISM-Band mit einer Schirmkabine ohne aktive Störunterdrückung auf der Empfangsseite zu nutzen, insbesondere wenn die Emissionen durch den Anregungspuls kritisch für eine Zulassung sind. Dies gilt auch für die nachfolgend beschriebenen Ausführungsformen, die eine Begrenzung bzw. Optimierung des Anregungspulses für das ISM-Band betreffen.

In einer denkbaren Ausführungsform des erfindungsgemäßen Magnetresonanztomographen weist der Magnetresonanztomograph eine Sendeantenne zum Aussenden eines Anregungspulses auf, wobei der Magnetresonanztomograph nichtlineare Bauelemente zum Verstimmen der Sendeantenne aufweist. Dies können beispielsweise PIN-Dioden, oder aber auch andere Dioden oder aktive Bauelemente wie Transistor oder FET sein. Dabei sind die nichtlinearen Bauelemente in einem von dem Patiententunnel für Hochfrequenz abgeschirmten Bereich des Magnetresonanztomographen angeordnet und der Filter für das ISM-Band bzw. ISM-Filter ist in der Signalverbindung zwischen nichtlinearem Bauelement und Antenne angeordnet, vorzugsweise in dem abgeschirmten Bereich.

Auf vorteilhafte Weise werden durch die Abschirmung der nichtlinearen Bauelemente von dem Patiententunnel und der Umgebung Emissionen von Frequenzanteilen vermieden, die durch die Nichtlinearität während des Anregungspulses von den zur Verstimmung verwendeten Bauelementen erzeugt werden und als Oberwellen nicht mehr in dem ISM-Band liegen. Der Filter verhindert, dass über die Signalverbindung zwischen nichtlinearem Bauelement und Sendeantenne Oberwellen übertragen werden. So trägt die Anordnung der nichtlinearen Bauelemente zur Einhaltung für Abstrahlungsgrenzwerte bei und ermöglicht bzw. vereinfacht den Verzicht auf eine Abschirmung des ganzen Magnetresonanztomographen durch eine Hochfrequenz-Kabine.

In einer möglichen Ausführungsform des erfindungsgemäßen Magnetresonanztomograph weist der Magnetresonanztomograph eine Hochfrequenzeinheit mit einem Vorverzerrer auf. Der Vorverzerrer ist ausgelegt, einen Anregungspuls zur Anregung der Kernspins derart zu vorzuverzerren, dass Signalanteile des Anregungspulses beim Aussenden, d.h. insbesondere nach der Verstärkung durch einen Hochfrequenzleistungsverstärker, außerhalb des ISM-Bandes gegenüber einem Anregungspuls ohne Vorverzerren reduziert sind. Denkbar ist es beispielsweise, dass der Vorverzerrer Signalanteile erzeugt und zumischt, die nach einer Verstärkung durch die Hochfrequenzeinheit den durch die Nichtlinearität der Hochfrequenzeinheit aus dem Anregungspuls erzeugten Oberwellen entsprechen, aber umgekehrtes Vorzeichen aufweisen und so die Oberwellen reduzieren oder auslöschen. Der Vorverzerrer kann dabei beispielsweise in einer digitalen Signalerzeugung realisiert sein oder auch durch analoge Bauelemente entsprechende Signale aus einem Eingangssignal für einen Leistungsverstärker erzeugt werden. Der Vorverzerrer kann dabei auch adaptiv sein, beispielsweise in seiner Charakteristik von einer Beladung des Patiententunnels abhängig gesteuert sein. Denkbar ist auch eine teilweise Regelung durch eine schnelle Rückkopplung von einem Sensor im Sendepfad, wie z.B. einem Richtkoppler.

Auf vorteilhafte Weise reduziert der Vorverzerrer Oberwellen außerhalb des ISM-Bandes und erleichtert bzw. ermöglicht so die Einhaltung der Abstrahlungsgrenzwerte auch ohne Abschirmkabine.

In einer denkbaren Ausführungsform des erfindungsgemäßen Magnetresonanztomographen ist eine Grenzfrequenz für eine Ausbreitung einer Radiowelle in dem Patiententunnel größer als eine Larmorfrequenz des Magnetresonanztomographen. Als Grenzfrequenz wird die Frequenz angesehen, bei der sich in dem Patiententunnel als Hohlleiter noch eine sich in LängsRichtung (z-Richtung) durch den Patiententunnel ausbreitende Radiowelle ausbilden kann. Die Grenzfrequenz wird auch als Cut-Off-Frequenz für einen Wellenleiter bezeichnet, hier für den Patiententunnel als Hohlleiter.

Liegt die Frequenz eines Radiosignals darunter, so erfolgt auf vorteilhafte Weise ein exponentieller Abfall der Feldstärke eines von außen kommenden Störsignals mit dem Abstand von der Öffnung im Inneren des Patiententunnels, sodass im Untersuchungsbereich (Field of View, FoV) das Störsignal wesentlich reduziert ist.

In einer möglichen Ausführungsform des erfindungsgemäßen Magnetresonanztomographen ist die zweite Empfangsantenne an einer Öffnung des Patiententunnels oder der Patientenliege angeordnet. Beispielsweise kann die zweite Empfangsantenne unmittelbar an einem Rand einer Öffnung angeordnet sein oder unter der Liegefläche für den Patienten.

Insbesondere, wenn die Frequenz des Störsignals unter der Grenzfrequenz für eine freie Welle liegt, bilden der Patient und der Patiententunnel einen Koaxialleiter für das Störsignal aus. Eine zweite Empfangsantenne in der Nähe des Patienten und der Öffnung kann auf vorteilhafte Weise das durch den Patienten in den Patiententunnel eingekoppelte Störsignal erfassen und so eine besonders effektive Unterdrückung durch den Empfänger ermöglichen.

In einer möglichen Ausführungsform weist der Magnetresonanztomograph einen Wellenleiter auf, der den Magnetresonanztomographen umgibt, wobei der Wellenleiter eine Grenzfrequenz bzw. Cut-Off-Frequenz aufweist, die größer als die Larmorfrequenz des Magnetresonanztomographen ist. Als Wellenleiter wird dabei jede elektrisch leitende Struktur angesehen, die den Magnetresonanztomographen zumindest in vier Raumrichtungen außenumfänglich umgibt, beispielsweise in Form einer Röhre oder eines Prismas, und durch ihre Leitfähigkeit bei der Larmorfrequenz des Magnetresonanztomographen eine Ausbreitung von Radiowellen bzw. elektrischen Feldern durch die leitende Struktur hindurch im Wesentlichen unterdrückt. Mit anderen Worten, auf einer von dem Magnetresonanztomographen abgewandten Seite der elektrisch leitenden Struktur des Wellenleiters ist ein Signal mit Larmorfrequenz gegenüber einem Signal auf der dem Magnetresonanztomographen zugewandten Seite um mehr als 30 dB, 40 dB, 60 dB oder mehr gedämpft.

Denkbar ist es auch, dass den erfindungsgemäßen Magnetresonanztomographen in einer Ausführungsform ein erfindungsgemäßer Wellenleiter umgibt oder auch eine klassische Schirmkabine. Eine hochfrequenzdichte Tür zu der Schirmkabine bzw. dem Wellenleiter ist dabei jedoch durch einen elektrisch leitenden Tunnel ersetzt, der wiederum einen Wellenleiter mit einer Cut-Off-Frequenz größer der Larmorfrequenz darstellt.

Durch die Dämpfung der Wechselfelder in dem Tunnel wird eine Abstrahlung in die Umgebung reduziert, sodass auf eine teure und in der Anwendung umständliche und fehleranfällige HFdichte Türe verzichtet werden kann, insbesondere bei den höheren Grenzwerten für Frequenzen in ISM-Bändern.

Auch hier ist grundsätzlich eine Verwendung eines Wellenleiters als Tür bzw. Abschirmung auch ohne aktive Störunterdrückung im Empfangsweg denkbar.

In einer denkbaren Ausführungsform des erfindungsgemäßen Magnetresonanztomographen weist der Wellenleiter eine elektrische leitende Verbindung mit dem Patiententunnel auf. Dabei ist es denkbar, dass der Wellenleiter gemeinsam mit dem Patiententunnel einen durchgehenden bzw. zusammenhängenden Wellenleiter ausbildet. Es ist auch möglich, dass der Wellenleiter an beiden Enden des Patiententunnels elektrisch mit dem Patiententunnel verbunden ist oder zwei Wellenleiter an entgegengesetzten Enden des Patiententunnels mit diesem elektrisch leitend verbunden sind. Dabei kann der Wellenleiter auch eine Grenzfrequenz bzw. Cut-Off-Frequenz aufweisen, die unterschiedlich von der Grenzfrequenz des Patiententunnels, jedoch ebenfalls oberhalb der Larmorfrequenz des Magnetresonanztomographen ist.

In einer denkbaren Ausführungsform des erfindungsgemäßen Magnetresonanztomographen weist die zweite Empfangsantenne eine im Wesentlichen Rundum-Empfangscharakteristik auf. Als im Wesentlichen Rundum-Empfangscharakteristik wird eine Empfindlichkeitsverteilung der Empfangsantenne in alle Raumrichtungen angesehen, bei der die Unterschied zwischen einer maximalen Empfindlichkeit und einer minimalen Empfindlichkeit in Abhängigkeit von den unterschiedlichen Richtungen kleiner als 6 dB, 12 dB, 18 dB oder 24 dB ist. Dies gilt vorzugsweise auch für unterschiedliche Polarisationen des Störsignals

Das Störsignal kann aus unterschiedlichen Richtungen und mit unterschiedlichen Polarisationen einfallen, sodass eine Antenne mit Richtcharakteristik oder Vorzugspolarisation nicht alle Störsignale erfassen kann. Eine Antenne mit Rundum-Empfangscharakteristik hingegen kann auf vorteilhafte Weise alle Störsignale erfassen.

In einer möglichen Ausführungsform des erfindungsgemäßen Magnetresonanztomographen weist der Magnetresonanztomograph eine Mehrzahl an zweiten Antennen auf und der Empfänger ist ausgelegt, das Störsignal in dem Magnetresonanzsignal in Abhängigkeit von Empfangssignalen der Mehrzahl der zweiten Empfangsantenne zu unterdrücken. Vorzugsweise sind die Mehrzahl der zweiten Empfangsantennen zueinander beabstandet angeordnet, beispielsweise in einem Abstand größer als ein Viertel einer Wellenlänge oder einer halben Wellenlänge einer Radiowelle mit der Larmorfrequenz.

Auch eine Mehrzahl an räumlich verteilten Antennen ist auf vorteilhafte Weise geeignet, eine bzw. mehrere Störquellen besser zu erfassen und damit die Störunterdrückung zu verbessern.

In einer denkbaren Ausführungsform des erfindungsgemäßen Magnetresonanztomographen ist die Mehrzahl der Empfangsantennen in einer Symmetrieanordnung zu dem Patiententunnel angeordnet. Denkbar ist beispielsweise eine Anordnung an dem Rand der Öffnung des Patiententunnels an zwei gegenüberliegenden Punkten, auf den Eckpunkten eines regelmäßigen Polygons oder Polyeders.

Auf vorteilhafte Weise kann mittels der Symmetrie der Antennen auch eine Symmetriebeziehung der Parameter hergestellt und so das Optimierungsverfahren zur Reduzierung des Störsignals vereinfacht und/oder beschleunigt werden.

Erfindungsgemäß weist der Magnetresonanztomograph einen Störunterdrückungssender und eine Störunterdrückungsantenne auf. Die Störunterdrückungsantenne ist in einem Abstand zu dem Patiententunnel angeordnet. Der Störunterdrückungssender ist dabei ausgelegt, ein Signal in einem Frequenzbereich eines Anregungspulses des Magnetresonanztomographen derart zu erzeugen und über die Störunterdrückungsantenne auszugeben, dass in einem Bereich einer Umgebung des Magnetresonanztomographen durch destruktive Interferenz eine Feldstärke des Anregungspulses reduziert wird. Als reduziert wird eine Reduzierung der Feldstärke bzw. Dämpfung des Signals des Anregungspulses in dem Bereich um mehr als 6dB, 12 dB, 24 dB, 40 dB oder 60 dB angesehen.

Auf vorteilhafte Weise kann durch den Störunterdrückungssender und die Störunterdrückungsantenne die Feldstärke der durch den Anregungspuls in die Umgebung des Magnetresonanztomographen emittierten elektromagnetischen Wellen derart reduziert werden, insbesondere in Synergie mit den anderen vorgeschlagenen Maßnahmen, dass auch ohne eine RF-Kabine die gesetzlichen Grenzwerte eingehalten werden können.

In einer denkbaren Ausführungsform des erfindungsgemäßen Magnetresonanztomograph weist der Magnetresonanztomograph eine Vielzahl an Störunterdrückungsantennen auf, wobei die Störunterdrückungsantennen in einem Abstand zu dem Patiententunnel und relativ zueinander angeordnet sind. Die Abstände sind vorzugsweise kleiner als eine Wellenlänge einer freien Radiowelle mit Larmorfrequenz und/oder größer als ein Zehntel der Wellenlänge. Beispielsweise können die Störunterdrückungsantennen in einer Ebene um die Öffnung des Patiententunnels herum angeordnet sein. Der Störunterdrückungssender ist ausgelegt, Signale in einem Frequenzbereich eines Anregungspulses des Magnetresonanztomographen über die Störunterdrückungsantennen auszugeben, sodass in mehreren Bereichen einer Umgebung des Magnetresonanztomographen durch destruktive Interferenz eine Feldstärke des Anregungspulses reduziert wird.

Auf vorteilhafte Weise ist es mit mehreren Störunterdrückungsantennen und Ansteuersignalen des Störunterdrückungssenders möglich, die elektromagnetischen Felder in mehreren Bereichen zu reduzieren bzw. die Abstrahlung in mehreren Richtungen zu reduzieren oder im Idealfall auf null zu bringen. Die Anzahl der Richtungen ohne Abstrahlungen entspricht dabei Nullstellen des Abstrahlungsdiagramms eines Multipols. Eine resultierende elektromagnetische Welle aus Signal des Anregungspulses aus lokaler Sendeantenne und der Störunterdrückungsantennen ist damit ein Multipolfeld (z.B. Quadrupolfeld), das mit zunehmendem Abstand zu der Quelle auf vorteilhafte Weise wesentlich schneller als z.B. ein Dipolfeld abfällt und es so ermöglicht, Grenzwerte für eine Abstrahlung auch ohne RF-Kabine einzuhalten.

In einer möglichen Ausführungsform des erfindungsgemäßen Magnetresonanztomographen ist der Störunterdrückungssender ausgelegt, die Signale für die Störunterdrückungsantenne bzw. Störunterdrückungsantennen durch Phasenverschiebung und/oder Amplitudenanpassung in Abhängigkeit von einem oder mehreren Sendeentstörparametern zu erzeugen. Vorzugsweise wird aus dem Anregungspuls durch einstellbare Verstärker bzw. Abschwächer und Phasenverschiebungsglieder in dem Störunterdrückungssender das bzw. die jeweiligen Signale für die Störunterdrückungsantenne bzw. Störunterdrückungsantennen erzeugt. Die Amplitudenverhältnisse und Phasenverschiebungen können dabei die Sendeentstörparameter darstellen oder aus dem oder den Sendeentstörparametern abgeleitet werden, beispielsweise durch analytische Funktionen, Tabellen oder Iterationsverfahren.

Der Anregungspuls kann dabei beispielsweise über einen Sensor wie einen Richtkoppler in der Leitung zwischen HochfrequenzLeistungsverstärker und Sendeantenne oder eine Sensor-Antenne im Patiententunnel erfasst werden. Denkbar ist es auch, direkt aus den digitalen Daten des Anregungspulses durch Skalierung und Phasenverschiebung mit AD-Wandler und Verstärker die Signale für die Störunterdrückungsantennen zu erzeugen.

Auf vorteilhafte Weise vereinfachen Sendeentstörparameter durch eine Reduktion der Anzahl der Variablen eine nachfolgend angegebene Bestimmung der Einstellung des Störunterdrückungssenders und ermöglichen eine schnellere Anpassung an veränderte Bedingungen wie andere Anregungspulse oder Umgebung durch Patient oder Bedienpersonal.

In einer möglichen Ausführungsform des Magnetresonanztomographen werden die Sendeentstörparameter bei der Herstellung oder der Installation eingestellt.

In einer denkbaren Ausführungsform des erfindungsgemäßen Magnetresonanztomographen weist der Magnetresonanztomograph ein Kalibrierelement in einer Umgebung des Magnetresonanztomographen und eine Störunterdrückungssteuerung auf. Das Kalibrierelement ist vorzugsweise eine Antenne bzw. Sensor, mit dem eine elektrische und /oder magnetische Feldstärke eines elektrischen und/oder magnetischen Wechselfeldes mit einer Frequenz des Anregungspulses erfasst und an die Störunterdrückungssteuerung weitergeleitet werden kann. Die Störunterdrückungssteuerung ist ausgelegt, mittels des Kalibrierelements eine Feldstärke in einem Frequenzbereich eines Anregungspulses am Ort des Kalibrierelementes zu erfassen. Beispielsweise kann die Störunterdrückungssteuerung einen Kalibrierempfänger aufweisen. Die Störunterdrückungssteuerung ist weiterhin ausgelegt, in Abhängigkeit von der erfassten Feldstärke den Sendeentstörparameter derart einzustellen, dass eine Feldstärke des Anregungspulses in einer vorbestimmten Umgebung des Kalibrierelements reduziert ist. Beispielsweise kann die Störunterdrückungssteuerung eine Amplitude und/oder Phase des von der bzw.

den Störunterdrückungsantennen derart variieren bzw. optimieren, dass durch destruktive Interferenz eine Reduzierung und/oder ein lokales Minimum der Feldstärke am Ort des Kalibrierelements erzielt wird.

Auf vorteilhafte Weise kann durch die adaptive Einstellung des bzw. der Sendeentstörparameter auf eine veränderte Umgebung durch Personen oder Gerätschaften in der Umgebung des Magnetresonanztomographen reagiert werden, um die Grenzwerte für Emission auch unter wechselnden Bedingungen einzuhalten. Gegebenen Falls kann bei Überschreiten eines Grenzwertes die Aussendung unterbrochen und/oder eine neue Bestimmung des Parameters angestoßen werden.

In einer möglichen Ausführungsform des erfindungsgemäßen Magnetresonanztomograph weist die Störunterdrückungsantenne einen Hochfrequenzleistungsverstärker auf. Der Hochfrequenzleistungsverstärker ist dabei vorzugsweise ausgelegt, aus einem Ansteuersignal mit kleiner Hochfrequenzleistung, beispielsweise weniger als 10 mW, 50 mW oder 100 mW ein zur Unterdrückung von Streufeldern des Anregungspulses ausreichendes elektromagnetisches Wechselfeld über die Störunterdrückungsantenne zu erzeugen.

Der Hochfrequenzleistungsverstärker ermöglicht es gegenüber einer rein passiven Störunterdrückungsantenne diese mit einem dünnen, flexiblen Hochfrequenzkabel mit dem Störunterdrückungssender zu verbinden und so die Installation zu vereinfachen. In vorteilhaftem Zusammenwirken mit dem Patiententunnel als Wellenleiter und der Mehrzahl an Störunterdrückungsantennen ist dabei nur eine Leistung im Bereich weniger Watt erforderlich, sodass auch die lokal angeordneten Hochfrequenzleistungsverstärker klein und leicht ausfallen können, was die Installation vereinfacht.

Die erfindungsgemäße Sendeentstörung mittels destruktiver Interferenz kann dabei auch unabhängig von den anderen Merkmalen des erfindungsgemäßen Magnetresonanztomographen Anwendung finden, sofern dies in den durch die Ansprüche definierten Schutzumfang der Erfindung fällt. Beispielsweise ist eine Verwendung eines aktiven Störunterdrückungssenders in einer nicht durch die Ansprüche abgedeckten Variante auch ohne aktive empfangsseitige Störunterdrückung denkbar, insbesondere wenn die zulässige elektromagnetische Abstrahlung der begrenzende Faktor ist.

Besondere Synergien ergeben sich jedoch in ISM-Bändern durch die höheren zulässigen Grenzwerte, die in Verbindung mit einer empfangsseitigen Störunterdrückung einen Betrieb auch ohne geschlossene Abschirmkabine ermöglichen.

In einer denkbaren Ausführungsform des erfindungsgemäßen Verfahrens weist der Schritt des Einstellens des Parameters den Schritt einer zeitlichen Mittelung mit der Bildung eines zeitlichen Mittelwertes in Abhängigkeit von dem Störsignal auf. Beispielsweise kann die Amplitude und/oder Phase des Störsignals erfasst werden und über einen Tiefpass oder durch Mittelwertbildung über ein Fenster gemittelt werden, sodass der Parameter nur langsamen Änderungen folgt.

Auf vorteilhafte Weise führt die zeitliche Mittelung dazu, dass die Entstörung nicht durch kurzfristige, möglicherweise sporadische Einflüsse verfälscht wird und keine höherfrequenten Rauschanteile durch die Entstörung künstlich erzeugt werden.

In einer möglichen Ausführungsform des erfindungsgemäßen Verfahrens mit einem Magnetresonanztomographen mit Kalibrierelement weist der Schritt des Einstellens die folgenden Unterschritte auf:

In einem Schritt misst der Empfänger eine erste Transferfunktion zwischen einer erster Empfangsantenne und dem Kalibrierelement. Das Messen kann beispielsweise dadurch erfolgen, dass der Empfänger oder die Entstörsteuerung den Störunterdrückungssender über eine Signalverbindung anweist, ein Signal mit vorbestimmter Amplitude und/oder vorbestimmter Phase in einem Frequenzbereich des Magnetresonanzsignals und/oder in einem angrenzenden Frequenzbereich über die Kalibrierantenne auszusenden. In diesem Falle ist eine Signalverbindung zwischen Störunterdrückungssender und Kalibrierelement erforderlich. Der Empfänger kann dann das Signal über die erste Empfangsantenne empfangen und so eine erste Transferfunktion bestimmen. Dies kann auch gleichzeitig für mehrere erste Empfangsantennen geschehen. Grundsätzlich wäre es aber auch denkbar, die Transferfunktion zu erfassen, indem über die erste Empfangsantenne gesendet wird und über das Kalibrierelement empfangen wird.

In einem weiteren Schritt misst der Empfänger eine zweite Transferfunktion zwischen zweiter Empfangsantenne und dem Kalibrierelement. Dies kann auf die gleiche Weise wie zuvor beschrieben erfolgen. Erfolgt das Aussenden des Signals über das Kalibrierelement, so können auf vorteilhafte Weise beide Transferfunktionen gleichzeitig erfasst werden.

Vorzugsweise sind die zur Erfassung der ersten und zweiten Transferfunktionen ausgesendeten Signale so codiert, dass der Empfänger auf einfache Weise die Amplituden- und Phasenbeziehung ermitteln kann. Denkbar wäre beispielsweise eine Pseudozufalls-Code, der eine schnelle und sicher Autokorrelation der Signale erlaubt. Das Signal kann dabei in Amplitude, Frequenz und/oder Phase moduliert werden. Denkbar sind auch Spread-Spectrum-Modulationen, bei denen das Signal zur Ermittlung der Transferfunktion auch unterhalb der Rauschgrenze der MR-Signale bleiben kann und ein gleichzeitiges Aussenden während einer Erfassung eines MR-Signals möglich ist. Auf diese Weise kann permanent auf Änderungen in der Umgebung eingegangen werden. Die permanente Emission des Signals lässt sich auch erreichen, indem ein zu den MR-Signalen benachbarter Frequenzbereich genutzt wird. Dann müssen jedoch die unterschiedlichen Ausbreitungsbedingungen durch die unterschiedlichen Frequenzen bei der Bestimmung der Transferfunktionen berücksichtigt werden.

In einem weiteren Schritt wird der Entstörparameter, bzw. werden bei mehreren ersten und zweiten Empfangsantennen die Entstörparameter in Abhängigkeit von der gemessenen ersten Transferfunktion bzw. Transferfunktionen und zweiten Transferfunktion bzw. Transferfunktionen eingestellt, sodass ein Anteil eines von der bzw. den zweiten Empfangsantennen empfangenen Störsignals in einem von dem Empfänger über die erste Empfangsantenne empfangenen Signal reduziert wird. Dabei wird vorzugsweise auch weiterhin das von der bzw. den zweiten Empfangsantennen empfangene Störsignal berücksichtigt. Die Transferfunktionen finden dabei Anwendung, in welcher Form das Signal einer einzelnen Empfangsantenne berücksichtigt wird. Dies lässt sich beispielsweise erreichen, indem für das über eine Autokorrelation separierte Störsignal in dem empfangenen MR-Signal durch ein Variationsverfahren bzw. lineares Optimierungsverfahren durch die Entstörsteuerung die Entstörparameter so eingestellt werden, dass der Störsignalanteil minimiert wird. Dabei gehen die Transferfunktionen als vorgegebene Dämpfungen und Phasenverschiebungen zwischen Empfangsantennen und Empfänger ein. Für ein Kalibrierelement ist die Transferfunktion dabei genau genommen nur für eine Störquelle an einem bestimmten Ort bzw. Richtung gültig. Bei Verwendung ausreichend vieler und geeignet positionierter Kalibrierelemente zur Bestimmung der Transferfunktionen können jedoch auch von dem jeweiligen Ort des Kalibrierelements unabhängige Transferfunktionen bestimmt werden.

Auf vorteilhafte Weise ermöglicht es das Bestimmen der Transferfunktionen unter Verwendung des Kalibrierelementes die Empfangsentstörung auf unterschiedliche Bedingungen in der Umgebung, wie beispielsweise Position von Personen oder Gerätschaften und die dadurch veränderten Ausbreitungbedingungen zu reagieren und die Entstörung anzupassen.

In einer möglichen Ausführungsform des erfindungsgemäßen Verfahrens erfolgt der Schritt des Einstellens in einem Zeitraum einer Sequenz, in der kein Magnetresonanzsignal empfangen wird, insbesondere auch kein Signal zur Anregung der Spins gesendet wird. Innerhalb einer Sequenz zur Bilderfassungen gibt es Zeiträume, in denen keine Anregungspulse emittiert werden und auch keine Erfassung eines Magnetresonanzsignals für eine Bildgebung erfolgt. Vorzugsweise handelt es sich dabei auch um Zeiträume der Sequenz, in der das Untersuchungsobjekt bzw. der Patient kein nennenswertes Magnetresonanzsignal emittiert, d.h. das Signal ist im Pegel mindestens 12 dB, 24 dB, 36 dB 48 dB oder 60 dB unter einem maximalen Magnetresonanzsignal. In einem derartigen Zeitraum erfolgt in dieser Ausführungsform des Verfahrens der Schritt des Einstellens.

Die Entstörsteuerung des Empfängers ist entsprechend ausgelegt, den Schritt des Einstellens in einem derartigen Zeitraum auszuführen. Beispielsweise kann Sie ein Triggersignal von der Steuerung des Magnetresonanztomographen erhalten.

Auf vorteilhafte Weise kann ohne Magnetresonanzsignal das Einstellen der Parameter vereinfacht werden, beispielsweise indem eine Energie des von der ersten Empfangsantenne empfangenen Signals in Abhängigkeit von den Parametern minimiert wird. Selbst wenn das Störsignal sich zeitlich beispielsweise in der Amplitude ändert, so sind doch die eingestellten Parameter bei gleicher räumlicher Anordnung weiterhin gültig und wirksam.

In einer anderen möglichen Ausführungsform des erfindungsgemäßen Verfahrens ist es aber auch denkbar, dass die Einstellung des Parameters permanent, d.h. in kurzen Abständen von 1 ms, 10 ms oder 100 ms erfolgt oder insbesondere in Echtzeit mit einer Verzögerung von weniger als 10, 100 oder 500 Mikrosekunden erfolgt.

Auf vorteilhafte Weise erlaubt es die permanente Einstellung des Parameters in Echtzeit oder nahezu Echtzeit, schnell auf neu auftretende Störsignale zu reagieren und deren negativen Effekt auf die Bildgebung möglichst zu minimieren.

In einer weiteren denkbaren Ausführungsform weist der Empfänger einen Speicher auf und speichert das empfangene Magnetresonanzsignal und das empfangene Störsignal. Der Empfänger kann dabei im Sinne der Erfindung beispielsweise auch einen Bildauswerterechner umfassen. Es ist in einer Ausführungsform aber auch denkbar, dass der Empfänger im engeren Sinne, das heißt die zur Aufbereitung der empfangenen hochfrequenten Magnetresonanzsignale genutzten Einrichtungen, den Speicher aufweisen. Das Einstellen des Parameters und das Verarbeiten des Magnetresonanzsignals mit dem Störsignal in Abhängigkeit von dem Parameter zur Reduzierung des Störsignals durch den Empfänger zu einem Empfangssignal können dann mit einer Verzögerung zu dem Empfang erfolgen. Die Verzögerung kann beispielsweise die Dauer einer Echosequenz, einer Anregungssequenz oder auch einer ganzen Bilderfassungssequenz umfassen, beispielsweise mehr als 10 ms, 100 ms, 0,5 s 10 s oder auch mehrerer Minuten, Stunden oder prinzipiell auch Tage.

Auf vorteilhafte Weise ist es durch das Speichern möglich, bereits vorhandene Ressourcen des Magnetresonanztomographen mit zu nutzen oder wegen der nicht erforderlichen Echtzeitbearbeitung auch mit geringerer Rechenleistung die Störunterdrückung kostengünstiger bereitzustellen. Auch ermöglicht die nachträgliche Störunterdrückung einen Vergleich von Ergebnissen unterschiedlicher Parametereinstellungen und Unterdrückungsverfahren und somit eine Optimierung der Entstörung.

In einer denkbaren Ausführungsform des erfindungsgemäßen Verfahrens weist der Empfänger bzw. die Entstörsteuerung eine Autokorrelationsvorrichtung auf. Mittels der Autokorrelationsvorrichtung kann die Entstörsteuerung einen Anteil des Störsignals in dem Magnetresonanzsignal bestimmen, beispielsweise eine Amplitude und eine Phasenverschiebung.

In einer anderen möglichen Ausführungsform weist die Entstörsteuerung eine Schätzeinrichtung auf, die den Anteil des Störsignals beispielsweise durch ein Optimierungsverfahren ermittelt, bei dem der Störanteil im Magnetresonanzsignal durch Variation des oder der Parameter minimiert wird, wie Least Mean Square Root (LSR) oder ähnliche Verfahren.

Bei mehreren Parametern optimiert die Entstörsteuerung die Mehrzahl der Parameter so, dass ein möglichst großer Anteil des Störsignals reduziert wird. Dies kann insbesondere bei mehreren Störquellen oder Reflexionen von Vorteil sein.

Auf vorteilhafte Weise erlauben Autokorrelation oder Schätzung eine flexible Anpassung an unterschiedliche Störquellen.

In einer möglichen Ausführungsform des erfindungsgemäßen Verfahrens weist der Schritt des Einstellens der Parameter die folgenden Teilschritte auf:
In einem Teilschritt werden die empfangenen Magnetresonanzsignale in einen Bildraum transformiert. Dabei können die üblichen in der MRT-Bildgebung verwendeten Verfahren wie beispielsweise eine Fourier-Transformation genutzt werden, aber auch andere Verfahren wie Compressed Sensing sind denkbar.

In einem anderen Teilschritt des Verfahrens werden die Störsignale im Bildraum von den Magnetresonanzdaten getrennt. Beispielsweise kann dies durch Vergleich zweier benachbarter Volumina erfolgen oder durch zwei zu unterschiedlichen Zeitpunkten erfassten Bilddaten des gleichen Volumens. Während die Bilddaten gleich oder ähnlich sind, sind durch Störsignale erzeugte Bildartefakte wegen der fehlenden Korrelation deutlich unterschiedlich.

Es ist auch denkbar, dass der erfasste Bildraum bzw. das dazugehörige Volumen größer ist als das Untersuchungsobjekt. Es sind dann im Bildraum Bereiche erfasst, die kein Magnetresonanzsignal aufweisen, sondern lediglich Störsignale. Durch diese Segmentierung kann das Störsignal separiert und ermittelt werden.

In einem weiteren Teilschritt werden die im Bildraum separierten Störsignale zurück in einen Rohdatenraum bzw. k-Raum transformiert, beispielsweise wieder mit einer Fourier-Transformation.

In einem anderen Teilschritt werden die Parameter für die Unterdrückung der Störsignale aus den transformierten Störsignalen in dem Rohdatenraum bestimmt. Beispielsweise geben die Koordinaten im k-Raum Informationen zu Phase und Frequenz eines Störsignals an, sodass unter Berücksichtigung der Anordnung der ersten Empfangsantenne und zweiten Empfangsantenne sowie Dämpfungsfaktoren und Phasenverschiebung der Signalwege eine Dämpfung und Signalverzögerung bestimmt werden kann, nach deren Anwendung auf das Störsignal der zweiten Empfangsantenne in einem Summensignal mit dem Empfangssignal der ersten Empfangsantenne die Störung in dem Magnetresonanzsignal reduziert ist.

Es ist aber auch denkbar, dass die Störsignale unmittelbar im Bildraum unterdrückt werden, beispielsweise durch Ausblenden der entsprechenden Bilddaten.

Dies kann insbesondere genutzt werden, wenn die Bilddaten nicht in dem Untersuchungsbereich liegen oder durch bereits erfasste, ungestörte Daten aus diesem Bereich ersetzt werden können. Auch ist es denkbar, die gestörten Daten im Bildraum durch besondere Kennzeichnung, z.B. eine Farbe oder Helligkeitswert zu kennzeichnen, sodass durch Störung verursachte Bildartefakte nicht für Merkmale des Untersuchungsobjektes gehalten werden können.

Auf vorteilhafte Weise erlaubt das Erkennen der Störsignale im Bildraum eine Trennung bzw. Segmentierung von Magnetresonanzdaten und Störungen. Dadurch können die Störsignale auf einfache Weise separat erfasst werden und die Eigenschaften besser identifiziert werden, was zu einer besseren und effektiveren Unterdrückung führt.

In einer denkbaren Ausführungsform des erfindungsgemäßen Verfahrens erfolgen die Teilschritte des Transformierens, des Trennens, des Rücktransformierens und des Bestimmens der Parameter auf Zeilen von Daten der empfangenen Magnetresonanzsignale im Rohdatenraum.

Durch die Anwendung des erfindungsgemäßen Verfahrens auf einzelne Zeilen des Rohdatenraumes und deren Transformierte im Bildraum kann auf vorteilhafte Weise schneller als beispielsweise beim Erfassen einer ganzen Schicht der Parameter geändert werden und so schneller auf Änderungen der Störsignale reagiert werden. Eine Wiederholung der Erfassung für eine ganze Schicht kann so vermieden werden.

In einer möglichen Ausführungsform des erfindungsgemäßen Verfahrens überwacht der Empfänger in einem Schritt das Störsignal auf Veränderungen und passt bei einer Änderung in einem weiteren Schritt den Parameter an. Beispielsweise ist es durch eine Bewegung der Störquelle oder eines reflektierenden Gegenstandes in der Umgebung des Magnetresonanztomographen möglich, dass sich die Feldverteilung der Störquelle in Phase und Amplitude ändert. Der Empfänger kann dann derartige Änderungen des Störsignals detektieren und den oder die Parameter entsprechend anpassen, sodass beispielsweise das von der zweiten Empfangsantenne bzw. den zweiten Empfangsantennen empfangene Störsignal mit angepasster Verstärkung und/oder Phasenverschiebung zu dem MRT-Signal addiert wird. Dabei ist es denkbar, dass der Empfänger bei der Überwachung Schwellwerte berücksichtigt und erst ein Überschreiten eines Schwellwertes als Änderung angesehen wird. Ebenso ist es denkbar, dass der Empfänger eine zeitliche Mittelung vornimmt, um kurzzeitige Schwankungen auszublenden. Die Mittelung kann dabei beispielsweise Eigenschaften des Störsignals wie Amplitude, Phase, Frequenz und/oder Frequenzverteilung betreffen, um nicht auf kurzfristige Schwankungen zu reagieren und möglichst wenig zusätzliche Rauschanteile in das Magnetresonanzsignal durch die Störunterdrückung einzubringen. Denkbar ist es auch, beim Schritt des Einstellens den Parameter über einen Zeitraum zu mitteln bzw. mit einem Tiefpass zu filtern.

Auf vorteilhafte Weise erlaubt es eine Überwachung des Störsignals durch den Empfänger, auf Änderungen des Störsignals zu regieren und so über längere Zeit eine effektive Entstörung eines veränderlichen Störsignals sicherzustellen. Schwellwerte und Mittelung erlauben dabei, die Änderungen zu begrenzen und Schwingungen durch Instabilitäten oder Artefakte durch zu starke Kompensation zu vermeiden.

In einer möglichen Ausführungsform des erfindungsgemäßen Verfahrens speichert der Empfänger in einem Teilschritt ein erstes empfangenes Magnetresonanzsignal in einem Speicher. In einem anderen Teilschritt speichert der Empfänger ein zweites empfangenes Magnetresonanzsignal in einem Speicher. In einem weiteren Teilschritt vergleicht der Empfänger das erste empfangene Magnetresonanzsignal und das zweite empfangene Magnetresonanzsignal. Erkennt der Empfänger dabei eine Abweichung, die auf externe Störer zurückzuführen ist, so führt der Empfänger eine Störunterdrückungsmaßnahme aus bzw. signalisiert der Steuerung des Magnetresonanztomographen eine Störung, sodass diese die Störunterdrückungsmaßnahme anstößt. So können plötzliche Amplitudenunterschiede in Magnetresonanzsignalen dicht beieinander liegender Schichten oder Messungen der gleichen Schicht, zum Beispiel aus einer Kalibriermessung, möglicherweise Störsignale sein.

Auf vorteilhafte Weise können durch den Vergleich der Magnetresonanzsignale benachbarter Bereiche oder gleicher Bereiche zu unterschiedlichen Zeiten Störsignale erkannt werden. Dies ist auch in einer Anwendung ohne zweite Empfangsantenne zum Empfang des Störsignals denkbar.

In einer denkbaren Ausführungsform des erfindungsgemäßen Verfahrens ist die Störunterdrückungsmaßnahme das Verwerfen des ersten und/oder zweiten empfangenen Magnetresonanzsignals. Denkbar ist auch dass zusätzlich oder alternativ eine Erfassung des ersten und/oder zweiten Magnetresonanzsignals wiederholt wird. Die Störunterdrückungsmaßnahme kann auch ein Einstellen des Parameters umfassen.

Durch die vorgeschlagenen Störunterdrückungsmaßnahmen können nach Erkennen eines Störsignals Bildartefakte in den Magnetresonanzaufnahmen vermieden werden.

In einer möglichen Ausführungsform des erfindungsgemäßen Verfahrens zum Betrieb eines Magnetresonanztomographen weist der Magnetresonanztomograph einen Patiententunnel, eine erste Empfangsantenne zum Empfang eines Magnetresonanzsignals aus einem Patienten in dem Patiententunnel, eine zweite Empfangsantenne zum Empfang eines Signals mit der Larmorfrequenz des Magnetresonanzsignals und einen Empfänger auf, wobei die zweite Empfangsantenne außerhalb des Patiententunnels oder in der Nähe einer Öffnung des Patiententunnels angeordnet ist. Das Verfahren weist den Schritt auf, ein Störsignal durch den Empfänger über die zweite Empfangsantenne zu empfangen. Durch die Anordnung der zweiten Empfangsantenne ist diese sensibler auf Signale außerhalb des Patiententunnels, die allein schon wegen der räumlichen Herkunft keine Magnetresonanzsignale sein können. Grundsätzlich kann das Signal der zweiten Empfangsantenne grundsätzlich auch geringe Anteile eines Magnetresonanzsignals aufweisen, die aber wegen ihres geringen Anteils bei der Störunterdrückung zunächst unberücksichtigt bleiben können oder mit in Unteransprüchen angegebenen Mitteln und Verfahren weiter reduziert bzw. vermieden werden können

In einem anderen Schritt empfängt der Empfänger ein Magnetresonanzsignal über die erste Empfangsantenne. Dabei wird hier als Magnetresonanzsignal das Signal bezeichnet, das primär zur Bilderfassung genutzt wird. Beispielsweise kann es sich bei den ersten Empfangsantennen um Lokalspulen am Körper des Patienten handeln. Das Magnetresonanzsignal kann Anteile des Störsignals enthalten, die mit der erfindungsgemäßen Vorrichtung bzw. Verfahren wie nachfolgend beschrieben weiter reduziert werden

In einem weiteren Schritt wird das empfangene Magnetresonanzsignals der ersten Empfangsantenne in Abhängigkeit von dem von der zweiten Antenne empfangenen Störsignals verworfen. Beispielsweise kann das von der zweiten Empfangsantenne empfangene Störsignal über einem Schwellpegel liegen, sodass trotz der Ausbreitungsdämpfung zwischen Ort der zweiten Empfangsantenne und Ort der ersten Empfangsantenne eine Störung einer aus dem Magnetresonanzsignal gewonnenen Abbildung zu erwarten ist. Der Empfänger oder die Steuerung des Magnetresonanztomographen kann dann das Signal der ersten Empfangsantenne verwerfen. Es ist dann denkbar, dass der Magnetresonanztomograph anschließend die Erfassung des verworfenen Signals wiederholt.

Auf vorteilhafte Weise kann durch die unterschiedliche Anordnung von erster und zweiter Empfangsantenne bereits anhand der Signalamplituden ein Störsignal erkannt werden.

In einer denkbaren Ausführungsform des erfindungsgemäßen Verfahrens zum Betrieb eines Magnetresonanztomographen mit einer Larmorfrequenz in einem ISM-Band weist das Verfahren den Schritt auf, einen Anregungspulse zur Anregung von Kernspins in einem Untersuchungsobjekt zu bestimmen. Das Bestimmen erfolgt dabei in Abhängigkeit von vorbestimmten Frequenzgrenzen des ISM-Bandes. Vorzugsweise wird dabei der Anregungspuls derart bestimmt, dass er spektrale Anteile außerhalb des ISM-Bandes nur unterhalb von vorbestimmten Grenzwerten aufweist. Die Begrenzung auf das ISM-Band kann beispielhaft durch die zu den nachfolgenden Ansprüchen beschriebenen Maßnahmen erreicht werden.

In einem anderen Schritt des Verfahrens sendet der Magnetresonanztomograph den Anregungspuls aus.

In einem weiteren Schritt des Verfahrens empfängt der Magnetresonanztomograph ein Magnetresonanzsignal und ermittelt in einem anderen Schritt eine Abbildung einer Verteilung von Kernspins in dem Untersuchungsobjekt. Die Abbildung kann anschließend auf einem Display ausgegeben werden.

Innerhalb des ISM-Bandes sind üblicherweise deutlich höhere Grenzwerte für in die Umgebung abgestrahlte Radiowellen zulässig. Anregungspulse können je nach Schichtdicke und Dauer so breitbandig werden, dass sie Anteile außerhalb des ISM-Bandes aufweisen, auch denn die Larmorfrequenz innerhalb des ISM-Bandes liegt. Wird dies durch nachfolgend erläuterte Maßnahmen vermieden, so kann auf zusätzliche Abschirmmaßnahmen wie eine RF-Kabine verzichtet werden, ohne die Grenzwerte zu überschreiten.

In einer möglichen Ausführungsform des erfindungsgemäßen Verfahrens weist der Schritt des Bestimmens eines Anregungspulses den Teilschritte auf, einen Anregungspulse zur Anregung der Kernspins in einer Schicht des Untersuchungsobjektes in Abhängigkeit von einer Relativposition der Schicht zu einer Magneteinheit, einer vorbestimmten Gradientenstärke, einer Dicke der Schicht und der Art der Messung zu bestimmen. Dies kann beispielsweise durch Verwendung parametrisierter Bibliotheken an Anregungspulsen erfolgen.

In einem weiteren Prüfschritt wird von dem Magnetresonanztomographen ermittelt, ob der Anregungspuls innerhalb der vorbestimmten Frequenzgrenzen liegt. Dies kann beispielsweise durch Spektralanalyse mittels FFT erfolgen. Wenn der Anregungspuls nicht innerhalb der vorbestimmten Frequenzgrenzen liegt, wird der Schritte des Ermittelns wiederholt. Dabei wird ein Pulsparameter variiert, der beim Ermitteln des Anregungspulses eine Auswirkung auf eine spektrale Frequenzverteilung des Anregungspulses hat.

Wenn der Anregungspuls innerhalb der vorbestimmten Frequenzgrenzen liegt, wird er in einem weiteren Schritt von dem Magnetresonanztomographen ausgesendet.

In einer denkbaren Ausführungsform des erfindungsgemäßen Verfahrens ist der Pulsparameter mit Auswirkung auf das Ermitteln des Anregungspulses einer aus den Parametern Dauer des Anregungspulse, Dicke der Schicht, Relativposition der Schicht oder Stärke der Gradienten. Mit anderen Worten, um einen anderen Anregungspuls zu erhalten, der innerhalb des ISM-Frequenzbandes mit der Larmorfrequenz des Magnetresonanztomographen liegt, können beispielsweise die Dauer des Anregungspulses, die Dicke der anzuregenden Schicht oder auch die Position der Schicht oder die Stärke der Gradienten verändert werden. Auch eine gleichzeitige Variation mehrerer Parameter ist denkbar.

Durch Variation eines oder mehrerer Pulsparameter kann auf vorteilhafte Weise ein Anregungspuls ermittelt werden, der die Grenzen des Frequenzbandes nicht überschreitet und so einen Betrieb im zulässigen Rahmen ermöglicht.

In einer möglichen Ausführungsform des erfindungsgemäßen Verfahrens weist der Schritt des Aussendens den Teilschritt des Veränderns der Relativposition des Untersuchungsobjektes zu der Magneteinheit vor dem Schritt des Ausstrahlen des Pulses auf.

Durch die Gradientenfelder kann es an den Rändern des FoV vorkommen, dass die Resonanzfrequenz der Kernspins stärker von der durch das B0-Feld bestimmten mittleren Larmorfrequenz des Magnetresonanztomographen abweicht. Durch Repositionierung der zu erfassenden Schicht mehr zum Isozentrum des B0-Feldes hin kann bei gleicher Bandbreite des Anregungspulses eine vollständige Anregung einer Schicht erzielt werden, ohne die Bandgrenzen des ISM-Bandes zu verlassen.

In einer möglichen Ausführungsform des erfindungsgemäßen Magnetresonanztomographen weist der Magnetresonanztomograph eine Steuereinheit zum Steuern der Bilderfassung auf, die insbesondere Parameter der Bilderfassung wie Zeitpunkt des Anregungspulses und/oder Zeitpunkt des Erfassens der Magnetresonanzsignale oder auch Frequenz des Anregungspulses und/oder Frequenzbereich des Empfängers beim Empfang des Magnetresonanzsignals beeinflussen kann. Weiterhin weist der Magnetresonanztomograph eine Schnittstelle in Signalverbindung mit der Steuereinheit auf. Die Schnittstelle kann wie nachfolgend erläutert eine Schnittstelle zum Datenaustausch mit anderen Magnetresonanzsystemen oder auch eine Hochfrequenzschnittstelle sein. Die Steuereinheit ist ausgelegt, eine Bilderfassung in Abhängigkeit von einem über die Schnittstelle von einem anderen Magnetresonanztomographen empfangenen Signal zu synchronisieren. Als Synchronisieren wird hierbei jegliche Aktivität gesehen, die eine wechselseitige Störung reduziert. Dies kann eine zeitliche Koordination, aber auch beispielsweise eine Änderung von Frequenzen umfassen.

Bei einem erfindungsgemäßen Magnetresonanztomographen kann die Steuereinheit auch ausgelegt sein, ein Signal mit einer Information über eine bevorstehende Bilderfassung an einen anderen Magnetresonanztomographen zu senden. Dieses Merkmal ergänzt vergleichbar einer Stecker-Steckdose-Kombination den vorhergehenden Magnetresonanztomographen, der ausgelegt ist, ein Signal von einem anderen Magnetresonanztomographen zu empfangen. Die Information betrifft vorzugsweise einen Zeitpunkt der Aussendung und/oder Frequenz eines Anregungspulses. Vorzugsweise ist ein erfindungsgemäßer Magnetresonanztomograph, wie nachfolgend erläutert, sowohl zum Senden als auch zum Empfangen eines Signals ausgelegt. Der Magnetresonanztomograph wird nachfolgend auch als erster Magnetresonanztomograph bezeichnet.

Der erfindungsgemäße erste Magnetresonanztomograph kann auch mit einem zweiten erfindungsgemäßen Magnetresonanztomographen in Signalverbindung gebracht werden. Der erste Magnetresonanztomograph und der zweite Magnetresonanztomograph weisen dazu jeweils eine Schnittstelle und eine Steuereinheit auf. Der erste Magnetresonanztomograph und der zweite Magnetresonanztomograph stehen über die Schnittstellen in Signalverbindung. Die Signalverbindung ermöglicht zumindest die Übertragung eines Signals von dem ersten Magnetresonanztomographen zu dem zweiten Magnetresonanztomographen, es ist aber auch ein bidirektionaler Informationsaustausch denkbar. Denkbar sind Punkt-zu-Punktverbindungen auf elektrischem, optischem oder drahtlosem Weg. Möglich sind als Signalverbindung auch Netzwerke, wie LAN, WAN, im speziellen TCP/IP. Die Steuereinheit des ersten Magnetresonanztomographen ist ausgelegt, eine Information zu einem bevorstehenden Bilderfassungsvorgang über die Schnittstelle an den zweiten Magnetresonanztomographen zu senden. Denkbar ist ein Zeitpunkt einer geplanten Aussendung eines Anregungspulses in absoluter Zeit oder relativ zu dem Signal, oder auch zur Frequenz bzw. einem Frequenzbereich des Anregungspulses. Die Steuereinheit des zweiten Magnetresonanztomographen ist dabei ausgelegt, die Information über die Schnittstelle zu empfangen und eine Bilderfassung in Abhängigkeit von der empfangenen Information auszuführen. Beispielsweise kann die Steuerung des zweiten Magnetresonanztomographen ausgelegt sein, eine Magnetresonanzsignalerfassung, d.h. eine Sequenz oder einen Teil davon, zeitlich zu verschieben, sodass der Anregungspuls des ersten Magnetresonanztomographen nicht stört. Der zweite Magnetresonanztomograph wird nachfolgend auch als anderer Magnetresonanztomograph bezeichnet.

Das erfindungsgemäße Verfahren zum Betrieb eines Magnetresonanztomographen kann auch zum Betrieb eines ersten Magnetresonanztomographen mit einer Steuereinheit zum Steuern der Bilderfassung und einer Schnittstelle in Signalverbindung mit der Steuereinheit erweitert werden. Das Verfahren weist dann den Schritt auf, ein Signal durch die Steuereinheit über die Schnittstelle von einem zweiten Magnetresonanztomographen zu empfangen. Dabei kann es sich um einen gezielten Informationsaustausch handeln, in dem der erste Magnetresonanztomograph über eine Datenschnittstelle eine Information bzw. Nachricht von dem zweiten Magnetresonanztomographen erhält, die Parameter wie Zeitpunkt und/oder Frequenz einer Beabsichtigten Bilderfassung aufweist. Denkbar ist aber auch, dass der erste Magnetresonanztomograph beispielsweise über einen Empfänger für Magnetresonanzsignale die Umgebung überwacht.

In einem anderen Schritt stellt die Steuereinheit des ersten Magnetresonanztomographen einen Parameter der Bilderfassung in Abhängigkeit von dem empfangenen Signal ein. Es ist beispielsweise denkbar, dass eine Sequenz zeitlich verschoben wird.

In einem weiteren Schritt wird durch den ersten Magnetresonanztomographen eine Bilderfassung gemäß dem eingestellten Parameter ausgeführt. Beispielsweise kann die Sequenz zu einem veränderten Zeitpunkt begonnen werden, sodass die Anregungspulse beider Magnetresonanztomographen gleichzeitig erfolgen oder der Anregungspuls des ersten Magnetresonanztomographen zu einem Zeitpunkt erfolgt, an dem der zweite Magnetresonanztomograph keine bildrelevanten Magnetresonanzsignale empfängt.

Auf vorteilhafte Weise ermöglichen der erfindungsgemäße Magnetresonanztomograph im Verbund mit einem zweiten Magnetresonanztomographen und das Verfahren zum Betrieb, wechselseitige Störungen von zwei Magnetresonanztomographen bei gleichzeitigem Betrieb zu reduzieren.

In einer möglichen Ausführungsform des erfindungsgemäßen Magnetresonanztomographen ist die Schnittstelle zum Datenaustausch ausgelegt ist. Mit anderen Worten, der erste Magnetresonanztomograph ist ausgelegt, über die Schnittstelle sowohl Daten an einen zweiten Magnetresonanztomographen zu versenden als auch über diese Schnittstelle Daten von einem zweiten Magnetresonanztomographen zu empfangen. Dabei ist die Steuereinheit ausgelegt, mittels Informationsaustausch über die Schnittstelle eine Bilderfassung mit einem zweiten Magnetresonanztomographen zu synchronisieren. Mit anderen Worten, die Steuereinheiten stimmen sich durch Nachrichten ab, wie die Bilderfassung erfolgt, sodass wechselseitige Störungen reduziert werden.

In einer denkbaren Ausführungsform des erfindungsgemäßen Magnetresonanztomographen weist das Signal eine Information zu einem Zeitpunkt und/oder Frequenz eines Sendevorganges auf. Die Zeit kann dabei beispielsweise absolut angegeben werden oder relativ zu dem Zeitpunkt des Versendens der Nachricht. Als Frequenzangabe kann eine Mittenfrequenz, und/oder eine Bandbreite, ein Frequenzbereich oder auch eine Kanalangabe erfolgen, die einen Frequenzbereich kodiert.

In einer möglichen Ausführungsform des erfindungsgemäßen Verfahrens zum Betrieb eines ersten Magnetresonanztomographen und eines zweiten Magnetresonanztomographen weist das Verfahren weiterhin den Schritt auf, eine Information über eine bevorstehende Bilderfassung des zweiten Magnetresonanztomographen durch eine Steuereinheit des zweiten Magnetresonanztomographen zu ermitteln und in einem weiteren Schritt ein Signal mit der Information an den ersten Magnetresonanztomographen zu senden.

Beispielsweise kann die Steuereinheit über die Schnittstelle eine Nachricht von einem zweiten Magnetresonanztomographen erhalten, dass dieser in 2 Sekunden einen Anregungspuls mit einer Mittenfrequenz gleich der Larmorfrequenz + 100 kHz mit einer Bandbreite von 200 kHz senden wird. Der erste Magnetresonanztomograph kann dann beispielsweise eine Sequenz vor einem eigenen Anregungspuls unterbrechen und weiterführen, nachdem der zweite Magnetresonanztomograph seine Sequenz beendet hat, oder den nächsten Anregungspuls gleichzeitig mit dem Anregungspuls des zweiten Magnetresonanztomographen senden. Auf vorteilhafte Weise kann so die gegenseitige Störung durch einen Anregungspuls des zweiten Magnetresonanztomographen während einer Empfangsphase vermieden werden.

In einer möglichen Ausführungsform des erfindungsgemäßen Magnetresonanztomographen weist das Signal eine Information zu einem Zeitpunkt und/oder Frequenz eines Empfangsvorganges auf. Die Information kann beispielsweise einen Beginn und eine Dauer und Frequenz angeben, wie dass der zweite Magnetresonanztomograph beginnend in einer Sekunde für 2 Sekunden auf einer Mittenfrequenz gleich der Larmorfrequenz minus 300kHz mit einer Bandbreite von 200 kHz empfangen wird. Der erste Magnetresonanztomograph kann dann beispielsweise eine Sequenz derart unterbrechen, dass er in dieser Zeit in dem genannten Frequenzband keinen Anregungspuls sendet.

Auf vorteilhafte Weise kann auch bei Empfang einer Nachricht zu einem geplanten Empfang in anderen Einheiten ein Aussenden des ersten Magnetresonanztomographen so verschoben werden, dass eine Störung reduziert wird.

In einer möglichen Ausführungsform ist es denkbar, dass die Steuereinheit des ersten Magnetresonanztomographen ausgelegt ist, die Frequenz eines Bilderfassungsvorganges in Abhängigkeit von der empfangenen Information zu ändern. Beispielsweise ist es denkbar, dass eine Bilderfassung unterschiedliche Schichten umfass, wobei diese durch einen Gradienten-Magnetfeld in z-Achse und damit in der effektiven Larmorfrequenz des Magnetresonanzsignals differenziert werden. Es ist so ein gleichzeitiger Betrieb mit reduzierter Wechselwirkung möglich, sofern die Reihenfolge der Schichten bei der Bilderfassung so geordnet wird, dass nie gleichzeitig in beiden Systemen eine Erfassung im gleichen Frequenzbereich erfolgt.

Auf vorteilhafte Weise ermöglicht die Differenzierung über die Frequenz eine gleichzeitige Erfassung von Magnetresonanzsignalen in benachbarten Magnetresonanztomographen und damit eine bessere Nutzung der Untersuchungszeit.

In einer denkbaren Ausführungsform des erfindungsgemäßen Magnetresonanztomographen weist der erste Magnetresonanztomograph als Schnittstelle einen Empfänger auf. Als Empfänger wird hier insbesondere ein Empfänger für Magnetresonanzsignale einschließlich einer Antenne wie Lokalspule oder Körperspule angesehen. Dabei ist der erste Magnetresonanztomograph ausgelegt, außerhalb einer Bilderfassung einen Anregungspuls eines zweiten Magnetresonanztomographen zu erfassen und die Bilderfassung in Abhängigkeit von dem erfassten Anregungspuls auszuführen. Beispielsweise ist es denkbar, dass der erste Magnetresonanztomograph dann zunächst Magnetresonanzsignale einer Schicht mit einer anderen effektiven Larmorfrequenz erfasst oder abwartet, bis eine Maximaldauer für eine Erfassung von Magnetresonanzsignalen in dem zweiten, den Anregungspuls aussenden Magnetresonanztomographen abgelaufen ist

Die Synchronisation kann so auf vorteilhafte Weise auch ohne Datenverbindung bzw. Änderungen in dem zweiten Magnetresonanztomographen erfolgen.

In einer möglichen Ausführungsform des erfindungsgemäßen Verfahrens zum Betrieb eines ersten Magnetresonanztomographen und eines zweiten Magnetresonanztomographen weist das Verfahren weiterhin den Schritt auf, eine Information über eine bevorstehende Bilderfassung des zweiten Magnetresonanztomographen durch eine Steuereinheit des zweiten Magnetresonanztomographen zu ermitteln und in einem weiteren Schritt ein Signal mit der Information an den ersten Magnetresonanztomographen zu senden.

Grundsätzlich ist es auch denkbar, dass die unterschiedlichen beschriebenen Maßnahmen miteinander kombiniert werden. Es könnte auch ein Protokoll für einen Informationsaustausch in zwei Magnetresonanztomographen vorgesehen sein, durch das die Bilderfassungen von beiden Systemen auf eine optimierte Weise miteinander verschachtelt werden, sodass sich die Erfassungsdauer nur geringfügig ändert, ohne dass wechselseitige Störungen auftreten. Im einfachsten Fall könnten dazu beispielsweise alle Anregungspulse gleichzeitig erfolgen, solange in beiden Magnetresonanztomographen im gleichen Zeitraum eine Bilderfassung stattfindet.

Bei der in Anspruch 1 definierten Variante weist der erfindungsgemäße Magnetresonanztomograph einen Patiententunnel, eine erste Empfangsantenne zum Empfang eines Magnetresonanzsignals aus einem Patienten in dem Patiententunnel, eine zweite Empfangsantenne zum Empfang eines Signals mit der Larmorfrequenz des Magnetresonanzsignals, und einen Empfänger auf, wobei die zweite Empfangsantenne außerhalb oder in der Nähe einer Öffnung des Patiententunnels angeordnet ist, wobei der Empfänger in Signalverbindung mit der ersten Empfangsantenne und der zweiten Empfangsantenne steht und der Empfänger ausgelegt ist, ein mit der zweiten Empfangsantenne empfangenes Störsignal in einem von der ersten Empfangsantenne empfangenen Magnetresonanzsignal zu unterdrücken.

Bei der in Anspruch 2 definierten Variante weist der erfindungsgemäße Magnetresonanztomograph einen Patiententunnel, eine erste Empfangsantenne zum Empfang eines Magnetresonanzsignals aus einem Patienten in dem Patiententunnel, eine zweite Empfangsantenne zum Empfang eines Signals nahe der Larmorfrequenz des Magnetresonanzsignals und einen Empfänger auf, wobei die zweite Empfangsantenne außerhalb oder in der Nähe einer Öffnung des Patiententunnels angeordnet ist, wobei der Empfänger in Signalverbindung mit der ersten Empfangsantenne und der zweiten Empfangsantenne steht und der Empfänger ausgelegt ist, ein mit der zweiten Empfangsantenne außerhalb eines Frequenzbereichs des Magnetresonanzsignals empfangenes breitbandiges Störsignal in einem von der ersten Empfangsantenne empfangenen Magnetresonanzsignal zu unterdrücken.

In einer möglichen Ausführungsform ist der erfindungsgemäße Magnetresonanztomograph ausgelegt, Magnetresonanzsignale mit einer Larmorfrequenz in einem Industrieband zu empfangen.

In einer denkbaren Ausführungsform weist der erfindungsgemäße Magnetresonanztomograph einen Sendepfad zum Aussenden eines Anregungspulses mit einem ISM-Filter auf, wobei der ISM-Filter ausgelegt ist, Signale außerhalb des ISM-Bandes zu unterdrücken.

In einer möglichen Ausführungsform weist der erfindungsgemäße Magnetresonanztomograph eine Sendeantenne zum Aussenden eines Anregungspulses auf, wobei der Magnetresonanztomograph nichtlineare Bauelemente zum Verstimmen der Sendeantenne aufweist, wobei die nichtlinearen Bauelemente in einem von dem Patiententunnel für Hochfrequenz abgeschirmten Bereich des Magnetresonanztomographen angeordnet sind, wobei der ISM-Filter zwischen nichtlinearem Bauelement und Antenne angeordnet ist.

In einer denkbaren Ausführungsform weist der erfindungsgemäße Magnetresonanztomograph eine Hochfrequenzeinheit auf, wobei die Hochfrequenzeinheit einen Vorverzerrer aufweist, der ausgelegt ist, einen Anregungspuls zur Anregung der Kernspins derart zu vorzuverzerren, dass ausgesendete Signalanteile des Anregungspulses außerhalb des ISM-Bandes gegenüber einem Anregungspuls ohne Vorverzerren reduziert sind.

In einer möglichen Ausführungsform des erfindungsgemäßen Magnetresonanztomographen ist eine Grenzfrequenz für eine Ausbreitung einer Radiowelle in dem Patiententunnel größer als eine Larmorfrequenz des Magnetresonanztomographen.

In einer denkbaren Ausführungsform des erfindungsgemäßen Magnetresonanztomographen weist der Magnetresonanztomograph einen Wellenleiter auf, der den Magnetresonanztomographen umgibt, wobei der Wellenleiter eine Grenzfrequenz aufweist, die größer als die Larmorfrequenz des Magnetresonanztomographen ist.

In einer möglichen Ausführungsform des erfindungsgemäßen Magnetresonanztomographen weist der Wellenleiter eine elektrische leitende Verbindung mit dem Patiententunnel auf.

In einer denkbaren Ausführungsform des erfindungsgemäßen Magnetresonanztomographen ist die zweite Empfangsantenne an einer Öffnung des Patiententunnels oder an der Patientenliege angeordnet.

In einer möglichen Ausführungsform des erfindungsgemäßen Magnetresonanztomographen weist die zweite Empfangsantenne eine Rundum-Empfangscharakteristik auf.

In einer denkbaren Ausführungsform des erfindungsgemäßen Magnetresonanztomographen weist der Magnetresonanztomograph eine Mehrzahl an zweiten Empfangsantennen auf und der Empfänger ist ausgelegt, das Störsignal in dem Magnetresonanzsignal in Abhängigkeit von Empfangssignalen der Mehrzahl an zweiten Empfangsantennen zu unterdrücken.

In einer möglichen Ausführungsform des erfindungsgemäßen Magnetresonanztomographen ist die Mehrzahl der zweiten Empfangsantennen in einer Symmetrieanordnung zu dem Patiententunnel angeordnet.

In einer denkbaren Ausführungsform des erfindungsgemäßen Magnetresonanztomographen weist der Empfänger eine Autokorrelationseinrichtung auf und die Autokorrelationseinrichtung ist ausgelegt, einen Anteil des von der zweiten Empfangsantenne empfangenen Signals in dem von der ersten Empfangsantenne empfangenen Magnetresonanzsignal zu bestimmen.

In einer möglichen Ausführungsform des erfindungsgemäßen Magnetresonanztomographen weist der Empfänger eine Schätzeinrichtung auf und die Schätzeinrichtung ist ausgelegt, einen Anteil des von der zweiten Empfangsantenne empfangenen Signals in dem von der ersten Empfangsantenne empfangenen Magnetresonanzsignal zu schätzen.

Erfindungsgemäß weist der Magnetresonanztomograph einen Störunterdrückungssender und eine Störunterdrückungsantenne auf, wobei die Störunterdrückungsantenne in einem Abstand zu dem Patiententunnel angeordnet ist, wobei der Störunterdrückungssender ausgelegt ist, ein Signal in einem Frequenzbereich eines Anregungspulses des Magnetresonanztomographen über die Störunterdrückungsantenne in Abhängigkeit von einem Sendeentstörparameter auszugeben, sodass in einem vorbestimmten Bereich einer Umgebung des Magnetresonanztomographen durch destruktive Interferenz eine Feldstärke des Anregungspulses reduziert wird.

In einer möglichen Ausführungsform des erfindungsgemäßen Magnetresonanztomographen weist der Magnetresonanztomograph ein Kalibrierelement in einer Umgebung des Magnetresonanztomographen und eine Störunterdrückungssteuerung auf, wobei die Störunterdrückungssteuerung ausgelegt ist, mittels des Kalibrierelements eine Feldstärke in einem Frequenzbereich eines Anregungspulses am Ort des Kalibrierelementes zu erfassen und in Abhängigkeit von der erfassten Feldstärke den Sendeentstörparameter derart einzustellen, dass eine Feldstärke des Anregungspulses in einer vorbestimmten Umgebung des Kalibrierelements reduziert ist.

In einer denkbaren Ausführungsform des erfindungsgemäßen Magnetresonanztomographen ist der Störunterdrückungssender ausgelegt, die Signale für die Störunterdrückungsantenne bzw. Störunterdrückungsantennen durch Phasenverschiebung und/oder Amplitudenanpassung in Abhängigkeit von einem oder mehreren Sendeentstörparametern zu erzeugen.

In einer möglichen Ausführungsform des erfindungsgemäßen Magnetresonanztomographen weist die Störunterdrückungsantenne einen Hochfrequenzleistungsverstärker auf.

In einer denkbaren Ausführungsform des erfindungsgemäßen Magnetresonanztomographen weist der Magnetresonanztomograph ein Kalibrierelement in einer Umgebung des Magnetresonanztomographen aufweist, wobei der Empfänger ausgelegt ist, eine erste Transferfunktion zwischen der ersten Empfangsantenne und dem Kalibrierelement sowie eine zweite Transferfunktion zwischen der zweiten Empfangsantenne und dem Kalibrierelement zu messen und in Abhängigkeit von der gemessenen ersten Transferfunktion und der zweiten Transferfunktionen den bzw. die Entstörparameter derart einzustellen, dass ein mit der zweiten Empfangsantenne empfangenes Störsignal in einem von der ersten Empfangsantenne empfangenen Magnetresonanzsignal reduziert ist.

Bei dem in Anspruch 6 definierten erfindungsgemäßem Verfahren zum Betrieb eines Magnetresonanztomographen, weist der Magnetresonanztomograph einen Patiententunnel, eine erste Empfangsantenne zum Empfang eines Magnetresonanzsignals aus einem Patienten in dem Patiententunnel, eine zweite Empfangsantenne zum Empfang eines Signals mit der Larmorfrequenz des Magnetresonanzsignals und einen Empfänger auf, wobei die zweite Empfangsantenne außerhalb des Patiententunnels oder in der Nähe einer Öffnung des Patiententunnels angeordnet ist, wobei das Verfahren die Schritte aufweist:

Empfangen eines Störsignals durch den Empfänger über die zweite Empfangsantenne;
Empfangen eines Magnetresonanzsignals durch den Empfänger über die erste Empfangsantenne;
Verarbeiten des Magnetresonanzsignals in Abhängigkeit von dem Störsignal durch den Empfänger zu einem Empfangssignal, wobei die Abhängigkeit von einem Parameter abhängt;
Einstellen des Parameters durch den Empfänger, sodass ein Anteil des Störsignals in dem Empfangssignal reduziert wird.

In der in Anspruch 7 definierten Variante des erfindungsgemäßen Verfahrens zum Betrieb eines Magnetresonanztomographen weist der Magnetresonanztomograph einen Patiententunnel, eine erste Empfangsantenne zum Empfang eines Magnetresonanzsignals aus einem Patienten in dem Patiententunnel, eine zweite Empfangsantenne zum Empfang eines Signals nahe der Larmorfrequenz des Magnetresonanzsignals und einen Empfänger auf, wobei die zweite Empfangsantenne außerhalb des Patiententunnels oder in der Nähe einer Öffnung des Patiententunnels angeordnet ist, wobei das Verfahren die Schritte aufweist:

Empfangen eines Frequenzanteils des Störsignals nahe der Larmorfrequenz durch den Empfänger über die zweite Empfangsantenne;
Empfangen eines Magnetresonanzsignals durch den Empfänger über die erste Empfangsantenne;
Verarbeiten des Magnetresonanzsignals in Abhängigkeit von dem Frequenzanteil des Störsignals durch den Empfänger zu einem Empfangssignal, wobei die Abhängigkeit von einem Parameter abhängt;

Einstellen des Parameters durch den Empfänger, sodass ein Anteil des Störsignals in dem Empfangssignal reduziert wird. In einer möglichen Ausführungsform des erfindungsgemäßen Verfahrens weist der Schritt des Einstellens des Parameters den Schritt einer zeitlichen Mittelung mit der Bildung eines zeitlichen Mittelwertes in Abhängigkeit von dem Störsignal auf.

In einer denkbaren Ausführungsform des erfindungsgemäßen Verfahrens zum Betrieb eines Magnetresonanztomographen weist das Verfahren die Schritte auf:

Messen einer Transferfunktion zwischen einer erster Empfangsantenne und dem Kalibrierelement;

Messen einer Transferfunktion zwischen zweiter Empfangsantenne und dem Kalibrierelement;

Einstellen des Entstörparameters in Abhängigkeit von den gemessenen Transferfunktionen derart, dass ein Anteil eines von der zweiten Empfangsantenne empfangenen Störsignals in einem von dem Empfänger über die erste Empfangsantenne empfangenen Signal reduziert ist.

In einer möglichen Ausführungsform des erfindungsgemäßen Verfahrens erfolgt der Schritt des Empfangens des Störsignals zu einem Zeitraum einer Sequenz, in der kein Magnetresonanzsignal zur Bildgebung empfangen wird.

In einer denkbaren Ausführungsform des erfindungsgemäßen Verfahrens weist der Empfänger einen Speicher auf und das Verfahren weist einen Schritt des Speicherns auf, bei dem der Empfänger das Störsignal sowie das Magnetresonanzsignal in dem Speicher speichert,

wobei der Schritt des Verarbeitens mit einer Verzögerung relativ zu dem Empfangen des Störsignals und/oder Magnetresonanzsignals erfolgt.

In einer möglichen Ausführungsform des erfindungsgemäßen Verfahrens weist der Empfänger eine Autokorrelationseinrichtung auf und in dem Schritt des Einstellens des Parameters bestimmt die Autokorrelationseinrichtung einen Anteil des Störsignals in dem Magnetresonanzsignal und der Parameter wird in Abhängigkeit von dem bestimmten Anteil des Störsignals eingestellt.

In einer denkbaren Ausführungsform des erfindungsgemäßen Verfahrens weist der Empfänger eine Schätzeinrichtung auf und in dem Schritt Einstellens des Parameters bestimmt die Schätzeinrichtung einen Anteil des Störsignals in dem Magnetresonanzsignal und der Parameter wird in Abhängigkeit von dem bestimmten Anteil des Störsignals eingestellt.

In einer möglichen Ausführungsform des erfindungsgemäßen Verfahrens weist der Schritt des Einstellens des Parameters die Unterschritte auf:

Transformieren der empfangenen Magnetresonanzsignale in einen Bildraum;

Trennen der Störsignale von den Magnetresonanzdaten; Transformieren der Störsignale in einen Rohdatenraum; Bestimmen der Parameter aus den transformierten Störsignalen in dem Rohdatenraum.

In einer denkbaren Ausführungsform des erfindungsgemäßen Verfahrens erfolgen die Schritte des Transformierens, des Trennens, des Rücktransformierens und des Bestimmens des Parameters auf Zeilen von Daten der empfangenen Magnetresonanzsignale im Rohdatenraum.

In einer möglichen Ausführungsform des erfindungsgemäßen Verfahrens überwacht der Empfänger in einem Schritt das Störsignal auf Veränderungen und passt bei einer Änderung in einem Schritt den Parameter an.

In einer denkbaren Ausführungsform des erfindungsgemäßen Verfahrens speichert der Empfänger in einem Schritt ein erstes empfangenes Magnetresonanzsignal in einem Speicher, in einem Schritt ein zweites empfangenes Magnetresonanzsignal und vergleicht in einem Schritt das erste empfangene Magnetresonanzsignal und das zweite empfangene Magnetresonanzsignal und führt bei einer Abweichung, die auf externe Störer zurückzuführen ist, eine Störunterdrückungsmaßnahme aus.

In einer möglichen Ausführungsform des erfindungsgemäßen Verfahrens ist die Störunterdrückungsmaßnahme eine aus Verwerfen des ersten und/oder zweiten empfangenen Magnetresonanzsignals, Wiederholen einer Erfassung des ersten und/oder zweiten Magnetresonanzsignals oder Einstellen des Parameters.

In einer möglichen Ausführungsform des erfindungsgemäßen Magnetresonanztomographen weist der Magnetresonanztomograph eine Steuereinheit zum Steuern der Bilderfassung und eine Schnittstelle in Signalverbindung mit der Steuereinheit auf, wobei die Steuereinheit ausgelegt ist, eine Bilderfassung in Abhängigkeit von einem über die Schnittstelle von einem zweiten Magnetresonanztomographen empfangenen Signal zu synchronisieren.

In einer denkbaren Ausführungsform des erfindungsgemäßen Magnetresonanztomographen weist der Magnetresonanztomograph eine Steuereinheit zum Steuern der Bilderfassung und eine Schnittstelle in Signalverbindung mit der Steuereinheit auf, wobei die Steuereinheit ausgelegt ist, ein Signal mit einer Information über eine bevorstehende Bilderfassung an einen zweiten Magnetresonanztomographen zu senden.

In einer möglichen Ausführungsform des erfindungsgemäßen Magnetresonanztomographen ist die Schnittstelle zum Datenaustausch ausgelegt, wobei die Steuereinheit ausgelegt ist, mittels Informationsaustausches über die Schnittstelle eine Bilderfassung mit einem zweiten Magnetresonanztomographen zu synchronisieren.

In einer denkbaren Ausführungsform des erfindungsgemäßen Magnetresonanztomographen weist das Signal eine Information zu einem Zeitpunkt und/oder Frequenz eines Sendevorganges auf.

In einer möglichen Ausführungsform des erfindungsgemäßen Magnetresonanztomographen weist das Signal eine Information zu einem Zeitpunkt und/oder Frequenz eines Empfangsvorganges auf.

Eine mögliche Ausführungsform der Erfindung weist ein Computerprogrammprodukt auf, welches direkt in einen Prozessor einer programmierbaren Steuerung eines erfindungsgemäßen Magnetresonanztomographen ladbar ist, mit Programmcode-Mitteln, um alle Schritte eines erfindungsgemäßen Verfahrens zum Betrieb eines Magnetresonanztomographen auszuführen, wenn das Programmprodukt auf der Steuerung ausgeführt wird.

Eine mögliche Ausführungsform der Erfindung betrifft ein computerlesbares Speichermedium mit darauf gespeicherten elektronisch lesbaren Steuerinformationen, welche derart ausgestaltet sind, dass sie bei Verwendung des Speichermediums in einer Steuerung eines erfindungsgemäßen Magnetresonanztomographen den Magnetresonanztomographen dazu veranlassen, das erfindungsgemäße Verfahren zum Betrieb eines Magnetresonanztomographen durchzuführen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Die in den Fig. 1 bis 5 und 7 bis 11 gezeigten Ausführungsformen fallen als solche nicht in den Schutzumfang der Ansprüche, da sie nicht alle Merkmale der Ansprüche 1 oder 2 bzw. 6 oder 7 enthalten.

Es zeigen:

Fig. 1      eine schematische Darstellung eines Magnetresonanztomographen ;

Fig. 2      eine schematische Darstellung des Empfängers und der ersten Empfangsantenne und der zweiten Empfangsantennen;

Fig. 3      eine schematische Darstellung eines Ablaufplans eines Teilaspekts eines erfindungsgemäßen Verfahrens;

Fig. 4      eine schematische Darstellung einer Hochfrequenzeinheit eines erfindungsgemäßen Magnetresonanztomographen;

Fig. 5      eine schematische Darstellung eines erfindungsgemäßen Magnetresonanztomographen umgeben von einem Wellenleiter;

Fig. 6      eine schematische Darstellung eines erfindungsgemäßen Magnetresonanztomographen mit einem Störunterdrückungssender;

Fig. 7      eine schematische Darstellung eines Ablaufplans eines Teilaspektes des erfindungsgemäßen Verfahrens;

Fig. 8      eine schematische Darstellung eines Ablaufplans eines Teilaspektes des erfindungsgemäßen Verfahrens;

Fig. 9      eine schematische Darstellung eines Ablaufplans eines Teilaspektes des erfindungsgemäßen Verfahrens;

Fig. 10      eine schematische Darstellung eines Ablaufplans eines Teilaspektes des erfindungsgemäßen Verfahrens;

Fig. 11      eine schematische Darstellung eines erfindungsgemäßen Magnetresonanztomographen, in Verbund mit weiteren Magnetresonanztomographen;

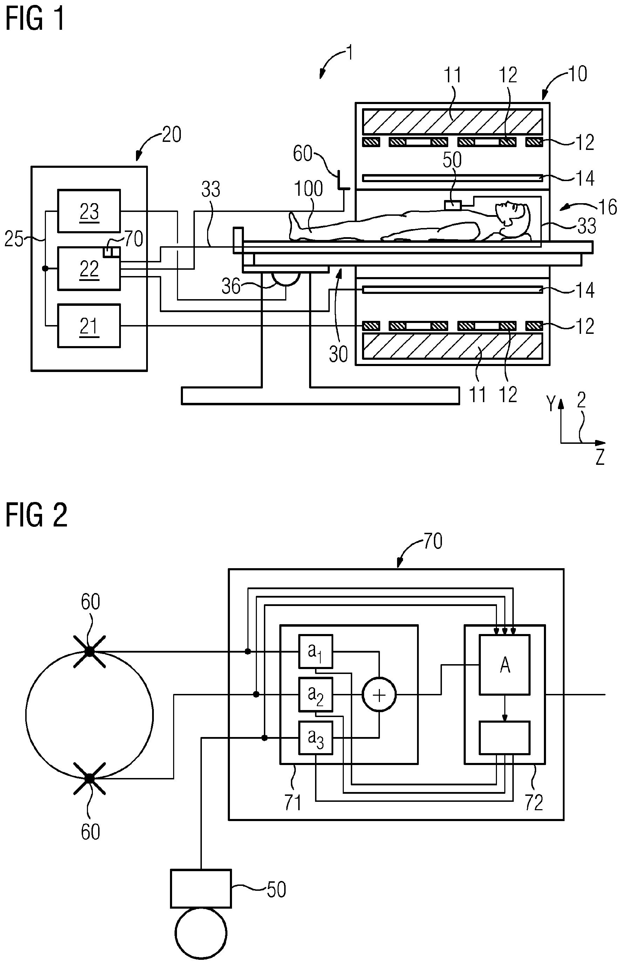

Fig. 1 zeigt eine schematische Darstellung einer Ausführungsform eines Magnetresonanztomographen 1 mit einer erfindungsgemäßen Lokalspule 50.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungspulse können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Auf dem Patienten 100 ist als eine erste Empfangsspule eine Lokalspule 50 angeordnet, die über eine Anschlussleitung 33 mit der Hochfrequenzeinheit 22 und deren Empfänger verbunden ist. Denkbar ist es aber auch, dass die Körperspule 14 eine erste Empfangsantenne im Sinne der Erfindung ist.

An einem Rand der Öffnung des Patiententunnels 16 sind vier zweite Empfangsantennen 60 angeordnet, die an den Ecken eines Quadrates angeordnet sind, das der kreisförmigen Öffnung einbeschrieben ist, sodass die Ecken auf dem Rand der Öffnung zu liegen kommen. Die vier zweiten Empfangsantennen 60 stehen in Signalverbindung mit dem Empfänger 70 der Hochfrequenzeinheit 22. Aufgrund der Mehrzahl der zweiten Empfangsantennen 60 ist es dabei denkbar, dass diese nicht alle eine Rundum-Empfangscharakteristik aufweisen, sondern beispielsweise Dipole sind und sich durch die unterschiedliche Ausrichtung zu einer Rundum-Charakteristik ergänzen. Es wäre aber beispielsweise auch denkbar, als einzige zweite Antenne mit Rundum-Charakteristik einen Kreuzdipol vorzusehen.

Es ist auch möglich, dass alternativ oder zusätzlich eine zweite Empfangsantenne 60 in der Patientenliege 30 angeordnet ist.

Der Patiententunnel hat dabei vorzugsweise einen Radius R für den gilt:

$$R < (Lambda_L * 1,841)/(2*Pi)$$

$Lambda_L$ gibt dabei die Wellenlänge einer Radiowelle in Luft bei der Larmorfrequenz des Magnetresonanztomographen 1 an. Ist der Radius R kleiner als der rechte Term, so breitet sich die Radiowelle in dem Patiententunnel 16 exponentiell gedämpft aus und das Störsignal ist in der Mitte im Untersuchungsbereich FoV stark gedämpft. $Lambda_L$ wird auch als Grenzwellenlänge eines Rundhohlleiters bezeichnet, die dazugehörige Frequenz als Grenzfrequenz.

Lediglich der Patient 100 wirkt durch seine endliche Leitfähigkeit als Seele eines Koaxialkabels, dessen Mantel die Wand des Patiententunnels 16 ist, und leitet ein bei den Beinen oder dem Kopfende eingekoppeltes elektromagnetisches Signal in den Untersuchungsbereich weiter. Die zweite Empfangsantenne 70 bzw. die zweiten Empfangsantennen 70 in der Nähe der Öffnung oder in der Patientenliege 30 nehmen dabei auf vorteilhafte Weise das von dem Patienten 100 in den FoV weitergeleitete Störsignal auf und machen dadurch die Kompensation in dem Empfänger 70 besonders effektiv.

Fig. 2 gibt eine schematische Darstellung der Funktionseinheiten einer möglichen Ausführungsform eines Empfängers 70 dar.

Die Summationseinrichtung 71 gewichtet die von der bzw. den ersten Empfangsantennen bzw. Lokalspule 50 und von den zweiten Empfangsantennen 60 eingehenden Signale mit Parametern, die auch komplex sein können, um eine Phasenverschiebung anzugeben. In einem analogen Empfänger 70 kann dies durch einen einstellbaren Verstärker in Verbindung mit einem einstellbaren Phasenschieber erfolgen. Der Realteil des Parameters entspricht dann dem Verstärkungsfaktor und der Imaginärteil der Phasenverschiebung. Anschließend werden in einer bevorzugten Ausführungsform die gewichteten Signale summiert, es sind aber auch andere nichtlineare Signaloperationen zur Kombination der Einzelsignale denkbar.

Eine Entstörsteuerung 72 erhält das kombinierte Signal als auch die einzelnen Signale der ersten Empfangsantenne und der zweiten Empfangsantennen 60. Um einen Anteil des Störsignals in dem kombinierten Signal zu bestimmen, ist es beispielsweise denkbar, dass die Entstörsteuerung 72 eine Autokorrelation der Signale vornimmt. Es ist aber auch denkbar, dass die Energie des kombinierten Signals bestimmt wird. In einer denkbaren Ausführungsform bestimmt die Entstörsteuerung 72 den Anteil des Störsignals in Abschnitten von Sequenzen des Magnetresonanztomographen, in denen kein Magnetresonanzsignal zur Bildgebung erwartet wird, sodass das kombinierte Signal nur das Störsignal aufweist. Dies kann beispielsweise in dephasierten Abschnitten einer Echosequenz der Fall sein, da sich die Amplituden einzelner Kernspins aufgrund unterschiedlicher Phase aufheben und in Summe kein Signal erzeugen.

Die Entstörsteuerung 72 optimiert dann beispielsweise nach einem Least-Square-Root-Verfahren (LSR) die Parameter in der Summationseinrichtung, sodass der Anteil bzw. die Energie des Störsignals in dem kombinierten Signal minimiert wird.

Grundsätzlich kann der Empfänger 70 sowohl in analoger Signalverarbeitungstechnik ausgeführt sein, sodass z.B. Verstärkungsregelung und Phasenverschiebung durch einen Parameter gesteuert und dann analog umgesetzt werden, oder auch als digitaler Empfänger, der entweder bereits digitalisierte Signale von der ersten Empfangsantenne und/ oder der zweiten Empfangsantenne 60 erhält oder diese bereits am Signaleingang mittels eines A/D-Wandlers digitalisiert.

Der Empfänger 70 leitet das kombinierte Signal, in dem das Störsignal weitestgehend unterdrückt ist, zur Bildgebung an die Steuerung 23 des Magnetresonanztomographen weiter.

Es ist dabei auch möglich, dass das Störsignal breitbandiger ist als das Magnetresonanzsignal. Die Anteile außerhalb des Frequenzbereichs des Magnetresonanzsignals korrelieren dabei üblicherweise mit den Anteilen innerhalb des Frequenzbereichs des Magnetresonanzsignals. Es kann daher gemäß der Erfindung auch hinreichend sein, wenn die zweite Empfangsantenne 60 das breitbandige Störsignal nur teilweise, beispielsweise in einem Frequenzbereich ungleich bzw. außerhalb des Frequenzbereichs des Magnetresonanzsignals empfängt. Ergänzend oder alternativ kann auch der Empfänger 70 ausgelegt sein, nur Frequenzen in diesem Frequenzbereich von der zweiten Empfangsantenne aufzunehmen. Der Empfänger 70 ist dabei ausgelegt, in Abhängigkeit von diesem Teil-Signal das Störsignal in einem von der ersten Empfangsantenne empfangenen Magnetresonanzsignal zu unterdrücken. Aufgrund der Korrelation kann beispielsweise die Amplitude der Frequenzanteile des Störsignals im Frequenzbereich des Magnetresonanzsignals mit einer Amplitude außerhalb des Frequenzbereichs verknüpft sein. Auf diese Weise ist es auch denkbar, dass die zweite Empfangsantenne 60 in Verbindung mit dem Empfänger 70 während einer Magnetresonanzmessung nur Frequenzanteile außerhalb des Frequenzbereichs des Magnetresonanzsignals auf Störsignale überwacht und in Abhängigkeit davon das Störsignal in den Signalen der ersten Empfangsantenne unterdrückt. Möglich wäre es dabei beispielsweise, dass der Empfänger 70 einen Zusammenhang zwischen Störsignal aus der ersten Empfangsantenne und Störsignalanteil aus der zweiten Empfangsantenne 60, auch als Transferfunktion bezeichnet, für ein Störsignal im Frequenzbereich der Magnetresonanzsignale zwischen Erfassungen von Magnetresonanzsignalen ermittelt und während der Erfassung von Magnetresonanzsignalen beispielsweise die Amplitude bzw. Skalierung anhand der von der zweiten Empfangsantenne außerhalb des Frequenzbereichs der Magnetresonanzsignale empfangenen Anteile des Störsignals anpasst.

In einer anderen denkbaren Ausführungsform des erfindungsgemäßen Magnetresonanztomographen (1) erfolgen die Unterdrückung des Störsignals in dem Empfangssignal nicht in Echtzeit, d.h. nicht unmittelbar bei Empfang des Störsignals und/oder des Magnetresonanzsignals, sondern das Magnetresonanzsignal und das Störsignal werden von dem Empfänger 70, der dabei auch Teile der Steuerung 23 des Magnetresonanztomographen 1 oder einer Bildauswertung umfassen kann, in einem Speicher gespeichert. Die nachfolgend zu dem Verfahren dargelegten Schritte erfolgen dann nicht mehr in Echtzeit oder nahezu in Echtzeit, sondern können auf den gespeicherten Daten mit Verzögerung ausgeführt werden, beispielsweise im Vorfeld einer Bildauswertung.

Dabei ist beispielsweise auch eine Kombination aus Empfang mit der zweiten Antenne außerhalb des Frequenzbandes der MR-Signale mit einer Echtzeit-Störunterdrückung oder einer Unterdrückung zu einem späteren Zeitpunkt, beispielsweise der Bildauswertung denkbar.

Die in Fig. 2 dargestellte Störunterdrückung in dem Empfänger kann auch mit einer einzelnen zweiten Antenne 60 ausgeführt werden. Umgekehrt ist es möglich, dass der Empfänger 70 mehrere Kanäle aufweist oder mehrere Empfänger 70 in dem Magnetresonanztomographen 1 vorgesehen sind, um die Magnetresonanzsignale mehrerer Lokalspulen 50 zu entstören. Dabei ist es denkbar, dass dabei die Signale der zweiten Empfangsantennen 60 von mehreren Empfängern 70 bzw. Kanälen der Empfänger 70 zur Störunterdrückung genutzt werden.

Es ist dabei sowohl denkbar, dass die Abhängigkeit zwischen dem von der zweiten Antenne empfangenen Störsignal und der Störunterdrückung linear ist als auch nicht-linear. Lineare Abhängigkeiten können dabei eine Phasenverschiebung um einen bestimmten Wert oder eine lineare Skalierung mit einem aus dem Signal der zweiten Antenne bestimmten Wert sein. Es ist aber auch denkbar, dass die Transferfunktion für das Störsignal auf dem Weg von erster Empfangsantenne und/oder zweiter Empfangsantenne Nichtlinearitäten aufweist, beispielsweise durch Mischer oder nichtlineare Verstärker, sodass auch auf das von der zweiten Empfangsantenne 60 empfangene Störsignal nichtlineare Operationen zur Störunterdrückung in dem Empfänger 70 angewendet werden müssen.

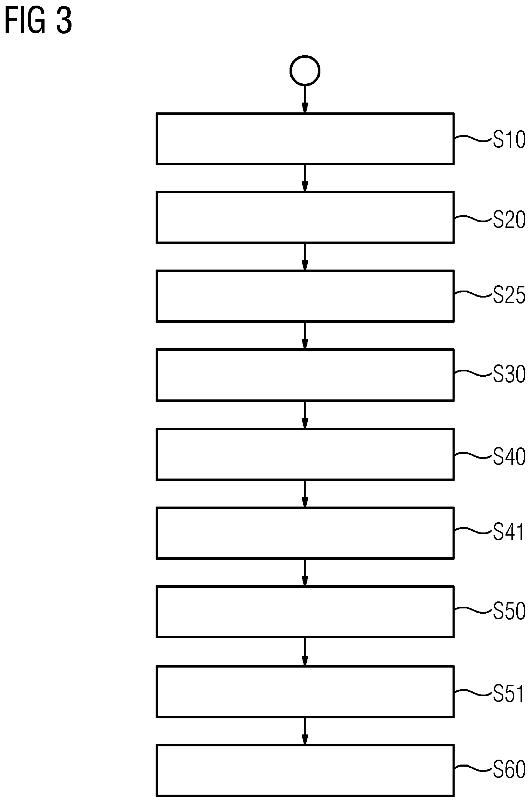

Fig. 3 zeigt einen schematischen Ablaufplan eines Teilaspekts eines erfindungsgemäßen Verfahrens.

In einem Schritt S10 empfängt der Empfänger 70 ein Störsignal über die zweite Empfangsantenne 60 bzw. über die Mehrzahl an zweiten Antennen 60. Das Störsignal wird über eine Signalverbindung zu dem Empfänger 70 übertragen werden. Dabei ist es auch denkbar, dass das Störsignal zunächst durch einen A/D-Wandler digitalisiert wird, bevor es zu dem Empfänger 70, in diesem Fall als digitaler Empfänger 70 ausgeführt, übertragen wird.

In einem Schritt S20 empfängt der Empfänger 70 ein Magnetresonanzsignal über die erste Empfangsantenne, beispielsweise die Lokalspule 50. Bei mehreren Lokalspulen 50 können entsprechend mehrere Empfänger 70 vorgesehen sein oder auch ein Empfänger 70 mit mehreren Kanälen mit jeweils einer Summationseinrichtung 71. Die Entstörsteuerung 72 kann dabei jeweils separat vorgesehen sein oder auch gemeinsam, was das nachfolgende Einstellen wegen ähnlicher Parameter für unterschiedliche Kanäle beschleunigen kann.

In einem Schritt S30 verarbeitet der Empfänger 70 das Magnetresonanzsignal in Abhängigkeit von dem Störsignal bzw. den Störsignalen bei mehreren zweiten Empfangsantennen 60 zu einem Empfangssignal. Beispielsweise werden das bzw. die Störsignale der zweiten Empfangsantenne bzw. zweiten Empfangsantennen 60 und das Magnetresonanzsignal der ersten Empfangsantenne mit unterschiedlichen Parametern gewichtet und verzögert und anschließend kombiniert. Das kann beispielsweise die Erzeugung einer Linearkombination sein. Das erzeugte Empfangs- bzw. Summensignal hängt dabei von einem bzw. mehreren Parametern ab.

In einem anderen Schritt S40 wird der Parameter bzw. die Parameter durch den Empfänger 70, insbesondere die Entstörsteuerung 72 derart eingestellt, dass ein Anteil des Störsignals in dem Empfangssignal reduziert wird. Wird beispielsweise das von der zweiten Empfangsantenne 60 empfangene Störsignal durch den eingestellten Parameter so skaliert, dass es gleiche Amplitude wie der über die erste Empfangsantenne Störsignalanteil aufweist und wird es mit einer Phasenverschiebung relativ dazu von 180 Grad versehen, so hebt sich das Störsignal in dem erzeugten Empfangssignal genau auf. Der Parameter bzw. die Parameter können dabei über Optimierungsverfahren wie beispielsweise Least Square Root (LSR) oder Wiener Filter ermittelt werden.

Der Schritt S40 kann dabei auch den Teilschritt S41 aufweisen, einen zeitlichen Mittelwert zu bilden und den Parameter zur Entstörung in Abhängigkeit von diesem Mittelwert einzustellen. Beispielsweise kann eine Amplitude oder Phase des Störsignals gemittelt werden, um statistische Schwankungen auszugleichen und weniger Rauschen in das Magnetresonanzsignal durch die Störunterdrückung einzubringen.

Es ist dabei auch denkbar, dass die Schritte S10 bis S30 jeweils auf empfangene Magnetresonanzsignale und empfangene Störsignale in Echtzeit ausgeführt werden, insbesondere bei analogen Empfängern 70. Es ist aber auch denkbar, dass die Schritte S10 bis S30 jeweils auf gespeicherte Störsignale und Magnetresonanzsignale ausgeführt werden, die beispielsweise für eine einzelne Sequenz oder einzelne Abschnitte davon digitalisiert werden.

In einer möglichen Ausführungsform des erfindungsgemäßen Verfahrens wird der Schritt S40 mit Störsignalen aus einem Zeitraum einer Sequenz ausgeführt, in der kein Magnetresonanzsignal zur Bildgebung empfangen wird. Beispielsweise können die Parameter mit Störsignalen der zweiten Empfangsantenne 60 und einem Signal der ersten Empfangsantenne in einem Zeitabschnitt von der Entstörsteuerung 72 bestimmt werden, in der die Kernspins dephasiert sind und kein Magnetresonanzsignal erzeugen. Es ist aber auch denkbar, dass in diesem Zeitabschnitt ohne Magnetresonanzsignal das Störsignal und das Signal der ersten Empfangsantenne lediglich digital erfasst und später ausgewertet wird.

Es ist dabei denkbar, dass in einem Schritt S50 die Entstörsteuerung 72 das Störsignal auf Veränderungen überwacht, beispielsweise unterschiedliche Amplitude, Frequenz oder Phase. Bei Erkennen einer derartigen Änderung oder überschreitet die Änderung einen vorbestimmten Schwellwert, so kann die Entstörsteuerung 72 die Schritte S10 des Empfangens von Störsignal und S20 Empfangen des Magnetresonanzsignals sowie das Einstellen eines Parameters zur Entstörung in Abhängigkeit von dem empfangenen Störsignal in einem Schritt S51 ändern, um die Entstörung an das geänderte Störsignal anzupassen.

Grundsätzlich ist es in einer Ausführungsform des erfindungsgemäßen Verfahrens auch denkbar, dass das empfangene Störsignal und/oder Magnetresonanzsignal von dem Empfänger 70 in einem Schritt S25 gespeichert wird. Der Empfänger 70 kann dabei auch Teile der Steuerung 23 des Magnetresonanztomographen 1 oder eines externen Bildauswerterechners umfassen. Die Schritte S20 bis S40 werden dabei nachträglich ausgeführt, beispielsweise am Ende einer Echosequenz, einer Anregungssequenz, einer Signalerfassung für eine Schicht des Untersuchungsobjektes oder auch nach Erfassung aller Daten.

Die Entkopplung der Signalerfassung von Störsignal und Magnetresonanzsignal von der Störunterdrückung ermöglichen es so auf vorteilhafte Weise, kostengünstigere Komponenten mit geringerer Rechenleistung einzusetzen oder auch vorhandene Ressourcen, z.B. aus der Bildauswertung doppelt zu nutzen.

Auch ist es dann denkbar, unterschiedliche Parametereinstellungen zu vergleichen und auszuwählen oder nachträglich zu optimieren. Auch kann die Anwendung auf Zeiträume mit Störungen begrenzt werden.

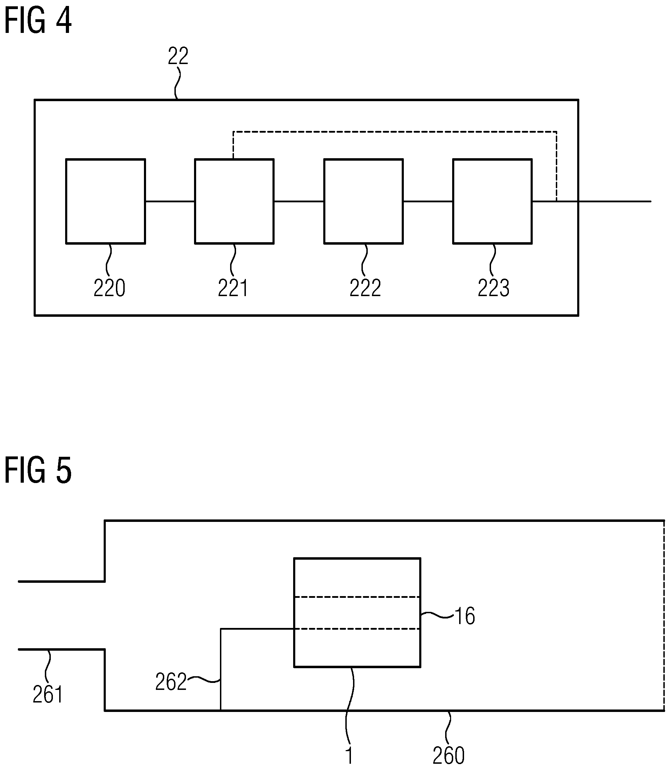

Fig. 4 zeigt eine schematische Darstellung einer Hochfrequenzeinheit eines erfindungsgemäßen Magnetresonanztomographen. Die Darstellung zeigt dabei nicht alle Details der Hochfrequenzeinheit.

Der Sendepfad der Hochfrequenzeinheit 22 weist dabei einen Pulserzeuger 220, einen Vorverzerrer 221, einen Leistungsverstärker 222 und einen ISM-Filter 223 auf.

Der Pulserzeuger 220 kann beispielsweise einen Oszillator, einen Modulator und einen Mischer aufweisen, mit dem einen Puls im Basisband erzeugt und dann auf die Larmorfrequenz umgesetzt wird.

Der Vorverzerrer 221 ist ausgelegt, einen Anregungspuls zur Anregung der Kernspins derart zu vorzuverzerren, dass Signalanteile des Anregungspulses außerhalb des ISM-Bandes gegenüber einem Anregungspuls ohne Vorverzerren reduziert sind. Denkbar ist es beispielsweise, dass der Vorverzerrer 221 Signalanteile erzeugt und zumischt, die nach einer Verstärkung durch den Leistungsverstärker den durch die Nichtlinearität des Leistungsverstärkers aus dem Anregungspuls erzeugten Oberwellen entsprechen, aber umgekehrtes Vorzeichen aufweisen und so die Oberwellen reduzieren oder auslöschen. Gleiches ist für Intermodulationen von Signalanteilen durch die Nichtlinearität denkbar. Der Vorverzerrer 221 kann dabei beispielsweise in einer digitalen Signalerzeugung realisiert sein oder auch durch analoge Bauelemente entsprechende Signale aus einem Eingangssignal für einen Leistungsverstärker erzeugt werden. Denkbar ist es auch, den Vorverzerrer 221 beispielsweise in einem digitalen Pulserzeuger 220 zu integrieren.

Das Ausgangssignal des Vorverzerrers 221 wird in dem Leistungsverstärker 222 verstärkt. Denkbar ist dabei ein Leistungsverstärker 22 mit linearer Kennlinie. Es ist aber auch möglich, dass der Vorverzerrer 222 das Eingangssignal des Leistungsverstärkers 222 gerade so verändert, dass nach der Verstärkung durch den Leistungsverstärker 222 ein Signal ohne unerwünschte Oberwellen erzeugt wird. Mit anderen Worten, die Kennlinie des Vorverzerrers 221 mit der Kennlinie des Leistungsverstärkers 222 multipliziert gibt idealerweise eine lineare Kennlinie, sodass das System aus Vorverzerrer 221 und Leistungsverstärker 222 Puls des Pulserzeugers ohne unerwünschte Oberwellen verstärkt.

Der Vorverzerrer 221 kann in einer Ausführungsform, wie in Fig. 4 durch die gestrichelte Linie vom Ausgang des ISM-Filters 223 dargestellt, auch adaptiv in dem Sinne sein, dass er durch Überwachung des Ausgangssignals des Leistungsverstärkers 222 die Vorverzerrung anpasst, sodass das Gesamtsystem aus Vorverzerrer und Leistungsverstärker eine lineare Charakteristik aufweist.

Im Anschluss an den Leistungsverstärker 222 wird das Signal vorzugsweise noch durch einen ISM-Filter 223 gefiltert. Der Filter unterdrückt dabei vorzugsweise Frequenzanteile außerhalb des ISM-Bandes, das der Magnetresonanztomograph 1 zur Bilderfassung nutzt. Beispielsweise kann es sich bei dem Filter um einen Bandpassfilter für das verwendete ISM-Band handeln, das Frequenzen außerhalb des ISM-Bandes um mehr als 12 dB, 24 DB, 40 dB oder 60 dB relativ zu einem Signal mit minimaler Dämpfung innerhalb des ISM-Bandes dämpft. Möglich ist aber auch ein Tiefpass. Je nach Ausführung der Hochfrequenzerzeugung des Magnetresonanztomographen kann der Filter beispielsweise zwischen Leistungsverstärker 222 und einem nicht dargestellten Hybridkoppler, zwischen Hybridkoppler und einer nicht dargestellten Sende-/Empfangs-Weiche oder zwischen Sende/Empfangs-Weiche und Sendeantenne angeordnet werden.

In einer Ausführungsform weist der Magnetresonanztomograph auch Verstimmelemente für die Sendeantenne auf. Dies können beispielsweise PIN-Dioden, oder aber auch andere Dioden oder aktive Bauelemente wie Transistor oder FET sein. Diese Verstimmelemente sind vorgesehen, um die Sendeantenne im Empfangsfall zu Verstimmen und Wechselwirkungen mit den Empfangsantennen zu vermeiden. Die Verstimmelemente weisen üblicherweise nichtlineare Kennlinien auf und können daher Oberwellen im Sendefall erzeugen. In einer bevorzugten Ausführungsform ist deshalb der ISM-Filter 223 zwischen den Verstimmelementen und der Sendeantenne angeordnet. Auch sind die nichtlinearen Bauelemente vorzugsweise in einem von dem Patiententunnel für Hochfrequenz abgeschirmten Bereich des Magnetresonanztomographen angeordnet.

So trägt die Anordnung der nichtlinearen Bauelemente zur Einhaltung für Abstrahlungsgrenzwerte bei und ermöglicht bzw. vereinfacht den Verzicht auf eine Abschirmung des ganzen Magnetresonanztomographen durch eine Hochfrequenz-Kabine.

In Fig. 5 ist eine Ausführungsform eines erfindungsgemäßen Magnetresonanztomographen 1, umgeben von einem Wellenleiter, schematisch dargestellt. Der Wellenleiter 260 kann dabei durch jede elektrisch leitende, den Magnetresonanztomographen 1 zumindest in 4 Raumrichtungen außenumfänglich umgebende Fläche bereitgestellt werden. Als elektrisch leitende Fläche wird dabei insbesondere eine metallische oder metallisierte Fläche oder Gewebe angesehen, die eine elektromagnetische Welle mit der Larmorfrequenz bei einem Durchgang um 60 dB, 80 dB, 100 dB oder mehr dämpft. Die Leitfähigkeit der Fläche kann dabei auch durch geometrische Untergliederung wie Schlitze auch anisotrop sein, solange die Leitfähigkeit parallel zum elektrischen Feldvektor des Wechselfeldes ausreicht, um die Dämpfung zu erzielen.

Vorzugsweise bildet die Fläche dabei den Wellenleiter 260 als einen Tunnel um den Magnetresonanztomographen in Form z.B. eines Zylinders, Quaders, Prismas mit einer Weite, die keine Ausbildung einer freien Welle mit Larmorfrequenz erlaubt. Bei einem Quader ist dies beispielsweise der Fall, wenn die längere Dimension des Querschnitts kleiner ist als eine halbe Wellenlänge einer elektromagnetischen Welle mit der Larmorfrequenz. Mit anderen Worten, die Cut-Off-Frequenz bzw. Grenzfrequenz des Wellenleiters 260 ist größer als die Larmor-Frequenz. Dadurch fällt das elektromagnetische Feld exponentiell mit dem Abstand von der Quelle ab, sodass aus dem Patiententunnel 16 herausleckende Wechselfelder schnell abfallen. Es ist dabei denkbar, dass der Wellenleiter 260 an einem oder beiden Enden offen ist, da durch den Abstand zu dem Patiententunnel 16 die exponentielle Dämpfung bereits stark genug ist, um die im ISM Band zulässigen Grenzwerte einzuhalten.

Denkbar ist es auch, dass der Magnetresonanztomograph 1 von einer Abschirmung umgeben ist, die größere Abmessungen als die halbe Wellenlänge aufweist. Es kann dann aber anstelle einer hochfrequenzdichten Türe eine tunnelförmige Zugangsöffnung 261 aus leitendem Material mit entsprechend kleinem Querschnitt zu dem Magnetresonanztomographen 1 vorgesehen sein, deren Abmessungen die freie Ausbreitung der Welle durch eine Cut-Off-Frequenz größer der Larmorfrequenz unterbinden. Die Zugangsöffnung 261 ist dabei vorzugsweise für Hochfrequenz elektrisch leitend mit der Abschirmung und/oder dem Wellenleiter verbunden. In einer Ausführungsform ist auch der Wellenleiter 260 mit dem Patiententunnel 16 für Hochfrequenz elektrisch leitend verbunden.

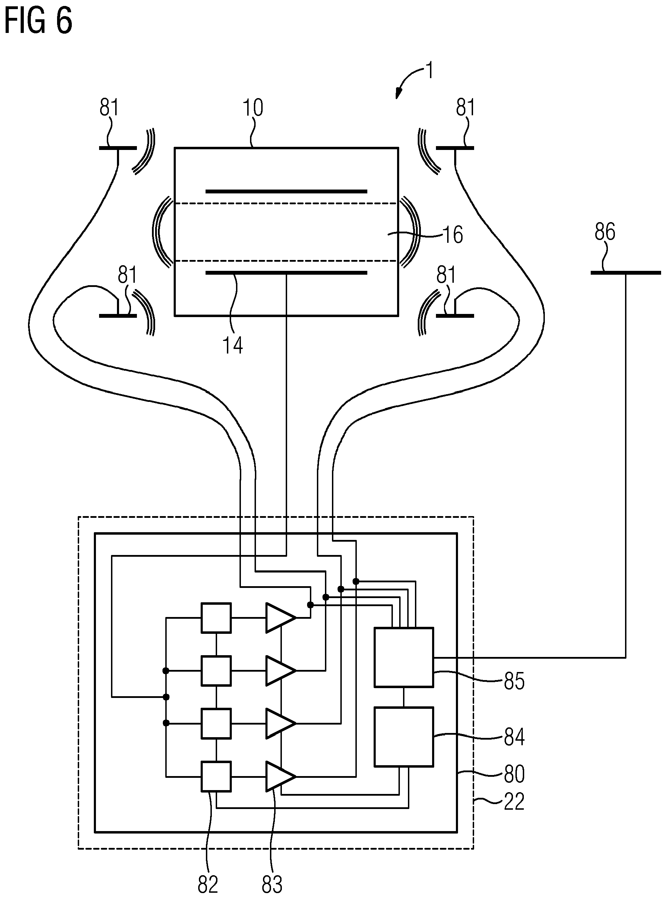

Fig. 6 zeigt eine schematische Darstellung einer Ausführungsform eines erfindungsgemäßen Magnetresonanztomographen 1 mit einem Störunterdrückungssender 80. Elektrische Wellen bzw. Wechselfelder lassen sich auch durch elektrische Felder mit gleicher Frequenz und Amplitudenbetrag, aber entgegengesetzter Polarität bzw. einer Phasenverschiebung um 180 Grad unterdrücken. Passen Amplitudenbeträge bzw. Phase nicht genau überein, so wird zumindest eine Reduzierung durch die destruktive Interferenz erzielt. Ein erfindungsgemäßer Magnetresonanztomograph 1 weist zur Erzeugung dieser Wechselfelder zur Störunterdrückung Störunterdrückungsantennen 81 auf, die um die Quelle der Felder, hier den Patiententunnel 16 herum angeordnet sind. Vorzugsweise decken die Störunterdrückungsantennen 81 alle Raumrichtungen um die Öffnung herum ab und es wird eine Symmetrie genutzt, wie z.B. gleiche Abstände zur Öffnung des Patiententunnels 16 und/oder eine Verteilung in gleichen Winkelabständen zu der Öffnung, um eine Ansteuerung der einzelnen Störunterdrückungsantennen 81 zu vereinfachen. Durch individuell für jede Störunterdrückungsantenne 81 einstellbare Amplitude und Phase ist aber auch eine beliebige Verteilung denkbar. Je nach Art des Wechselfeldes kann es sich dabei um Antennen mit vorzugsweise elektrischem Feld wie beispielsweise Dipole oder mit magnetischem Feld wie beispielsweise Sendespulen handeln. Die Ausrichtung der Antennen bzw. die Polarisation des erzeugten Feldes ist dabei vorzugsweise an den Feldrichtungen der zu unterdrückenden Wechselfelder orientiert.

Das Signal, das von den Störunterdrückungsantennen 81 ausgesendet wird, soll die Abstrahlung des Anregungspulses reduzieren und muss damit eine vorbestimmte Amplituden- und Phasenbeziehung zu dem Anregungspuls aufweisen. Vorzugsweise werden deshalb die Signale aus dem Anregungspuls analog oder auch aus der digitalen Pulserzeugung abgeleitet. Denkbar ist es aber auch, die Signale durch separate Einheiten unabhängig von der Pulserzeugung bereitzustellen, solange die notwendige Amplituden- und Phasenbeziehung hergestellt wird.

In Fig. 6 ist symbolisch eine Verbindungsleitung zwischen der Körperspule 14 als Quelle der elektromagnetischen Wellen und dem Störunterdrückungssender angegeben. Denkbar wäre eine direkte Verbindung über einen Leistungsteiler oder beispielsweise einen Richtkoppler, auch ein Sensor in dem Patiententunnel zur direkten Erfassung des elektromagnetischen Feldes wäre möglich. Es wäre aber auch möglich, ein Referenzsignal zur Erzeugung des Signals zur Störunterdrückung aus dem Leistungsverstärker 222 oder dem Pulserzeuger 220 zu entnehmen.

Das von dem Anregungspuls abgeleitete Referenzsignal für die Störunterdrückung wird anschließend durch einstellbare Phasensteller 82 für die einzelnen Störunterdrückungsantennen 81 verzögert bzw. in der Phase verschoben und anschließend durch einstellbare Verstärker 83 in der Amplitude verstärkt, bevor es über die Störunterdrückungsantennen 81 emittiert wird.

Die Einstellung der Phasensteller 82 und der Verstärker 83 erfolgt dabei durch eine Störunterdrückungssteuerung 84 über eine Signalverbindung. Dabei ist es denkbar, dass die Störunterdrückungssteuerung 84 vorbestimmte Phasenverschiebungen und Amplituden einstellt, die beispielsweise bei der Installation des Magnetresonanztomographen 1 ermittelt werden.

Es ist aber auch möglich, dass die Einstellung durch eine Kalibriermessung erfolgt. Dabei ist es denkbar, dass ein Kalibrierempfänger 85 mittels eines oder vorzugsweise mehrerer im Raum verteilter Kalibrierelemente 86 das zu unterdrückende Wechselfeld aufnimmt. Gleichzeitig erfasst der Kalibrierempfänger 85 die den Störunterdrückungsantennen 81 zugeführten Signale und übermittelt die erfassten Werte der Störunterdrückungssteuerung 84. Die Störunterdrückungseinstellung 84 kann dann beispielsweise die Störunterdrückungssteuerung 84 durch ein lineares Optimierungsverfahren wie LSR die Phasen und Amplituden der einzelnen Störunterdrückungsantenne derart einstellen, dass am Ort der Kalibrierantennen 86 die Feldstärke null wird. Sind die n Kalibrierelemente 86 über den Raumwinkel verteilt, so kann das resultierende Wechselfeld von Körperspule 14 und Störunterdrückungsantennen 81 zu einem Multipolfeld mit n Nullstellen bzw. Abstrahlkeulen verändert werden, die in hoher Potenz mit dem Abstand abnehmen und eine effektive Unterdrückung ermöglichen.

Grundsätzlich ist dabei die Ausbreitung der Felder umkehrbar. Zur Kalibrierung wäre es also auch denkbar, dass das bzw. die Kalibrierelemente 86 ein Signal aussenden und die Körperspule 14 und die Störunterdrückungsantennen 84 das Signal empfangen und dann die Störunterdrückungssteuerung 84 eine geeignete Phasenbeziehung und Amplituden ermitteln.

Darüber hinaus könnte das Kalibrierelement 86 auch dafür genützt werden, ein Referenzsignal für die Empfangsstörunterdrückung auszusenden. Das Referenzsignal müsste dabei so codiert bzw. moduliert werden, dass es von einem Magnetresonanzsignal durch den Empfänger 70 unterscheidbar ist. Das könnte beispielsweise auch unterhalb der Rauschgrenze des Magnetresonanzsignals mit einer Spread-Spectrum-Modulation erreicht werden. Denkbar wäre auch eine Aussendung in einem benachbarten Frequenzbereich. Es ist erforderlich, dass der Empfänger 70 dabei eine Korrelation zwischen dem Referenzsignal und den über die zweiten und ersten Empfangsantennen empfangenen Signale herstellen kann, um die Störunterdrückung zu optimieren. Auf diese Weise könnten beispielsweise Einstellungen zur Unterdrückung von Störsignalen aus bestimmten Richtungen bestimmt werden.

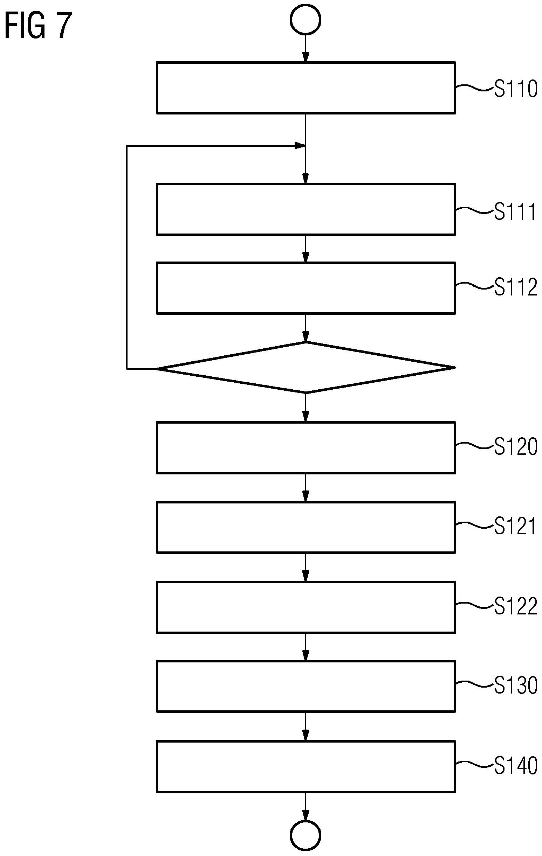

Fig. 7 zeigt schematisch einen Ablaufplan eines möglichen Teilaspekts des erfindungsgemäßen Verfahrens. In der Fig. 7 wird insbesondere der Aspekt betrachtet, wie ein Anregungspuls gestaltet und ausgesendet werden muss, um auch ohne Abschirmkabine regulatorische Grenzwerte für Abstrahlung von Hochfrequenz einzuhalten, insbesondere wenn die Larmorfrequenz in einem ISM-Band liegt. Die bereits zu Fig. 3 erläuterten Schritte eines erfindungsgemäßen Verfahrens sind in Fig. 7 unter dem Schritt S130 zusammengefasst und nicht noch einmal erläutert. Grundsätzlich ist es aber auch möglich, die in zu Fig. 7 erläuterten Maßnahmen zur Emissionsbeschränkung beim Anregungspuls auch ohne die Empfangsstörunterdrückung aus Fig. 3 zu implementieren.

In einem Schritt S110 wird ein Anregungspuls zur Anregung von Kernspins in einem Untersuchungsobjekt durch die Steuerung 23 bestimmt. Dazu wird zunächst in einem 5111 ein Anregungspuls zur Anregung der Kernspins in einer Schicht des Untersuchungsobjektes von der Steuerung 23 ermittelt. Dies kann beispielsweise in Abhängigkeit von der gewählten Sequenz bzw. Untersuchungsart durch Auswahl aus einer Bibliothek an Anregungspulsen erfolgen. Die Frequenz, die Dauer, die Leistung und die spektrale Verteilung hängen dabei von mehreren Parametern ab. Die Mittenfrequenz ergibt sich aus den zu erfassenden Kernspins, der Stärke des homogenen statischen Magnetfeldes B0, der Lage der Schicht in Bezug auf das Gradientenfeld sowie die Stärke des Gradientenfeldes. Die spektrale Verteilung und Bandbreite ergibt sich wiederum aus der Stärke des Gradientenfeldes und der Dicke der Schicht in Richtung des Feldgradienten. Die Amplitude wiederum hängt von der Dauer des Anregungspulses, dem anzuregenden Volumen und der erwünschten Anregungsstärke, auch als Flipwinkel bezeichnet ab. Dabei wird in Teilschritt S111 in Abhängigkeit von diesen Randbedingungen ein Satz von Parametern bestimmt, der einen möglichen Anregungspuls für diese Randbedingungen beschreibt. Denkbar ist, dass eine Bibliothek oder Tabelle unterschiedlicher Parametersätze für bestimmte Standardsituationen, wie zum Beispiel Bilderfassungen bestimmter Organe, vorgegeben ist und aus diesen ausgewählt wird.

In einem weiteren Teilschritt S112 wird von der Steuerung 23 überprüft, ob der ermittelte Anregungspuls innerhalb der vorbestimmten Frequenzgrenzen liegt. Im einfachsten Fall kann zum Beispiel mit Hilfe der Mittenfrequenz und der spektralen Frequenzverteilung die oberste und die unterste Frequenz des Anregungspulses errechnet werden. Denkbar ist es, dabei auch die Leistungsverteilung zu errechnen und Grenzwerte für eine frequenzabhängige Leistung zu bewerten.

Der Teilschritt S111 wird wiederholt, wenn bei der Bewertung festgestellt wird, dass der ermittelte Anregungspuls Grenzwerte überschreitet, insbesondere Grenzwerte für eine zulässige Abstrahlung von Hochfrequenzleitung. Dies betrifft vor allem bei einem Betrieb des Magnetresonanztomographen in einem ISM-Band eine Emission außerhalb des ISM-Bandes, die stärkeren Beschränkungen unterliegt.

Dabei werden bei einer Wiederholung des Teilschrittes S111 Parameter variiert, die darauf Einfluss nehmen. Ein längerer Puls kann beispielsweise mit einer kleineren Leistung die gleiche Anregung erzielen. Bei einem geringeren Gradienten ist eine geringere Frequenzbandbreite erforderlich, um die gleiche Schichtdicke anzuregen.

Hält der in Teilschritt S111 ermittelte Anregungspuls die Grenzwerte ein, so wird er in einem Schritt S120 des Verfahrens von der Hochfrequenzeinheit 22 ausgesendet.

In einem Schritt S130 wird, wie bereits zu Fig. 3 in einer beispielhaften Ausführungsform beschrieben, das Magnetresonanzsignal von dem Empfänger 70 empfangen.

Anschließend wird in einem Schritt S140 eine Abbildung einer Verteilung von Kernspins durch die Steuerung 23 aus dem empfangenen Magnetresonanzsignal ermittelt. Vorzugsweise wird die Abbildung abschließend auf einem Display wiedergegeben.

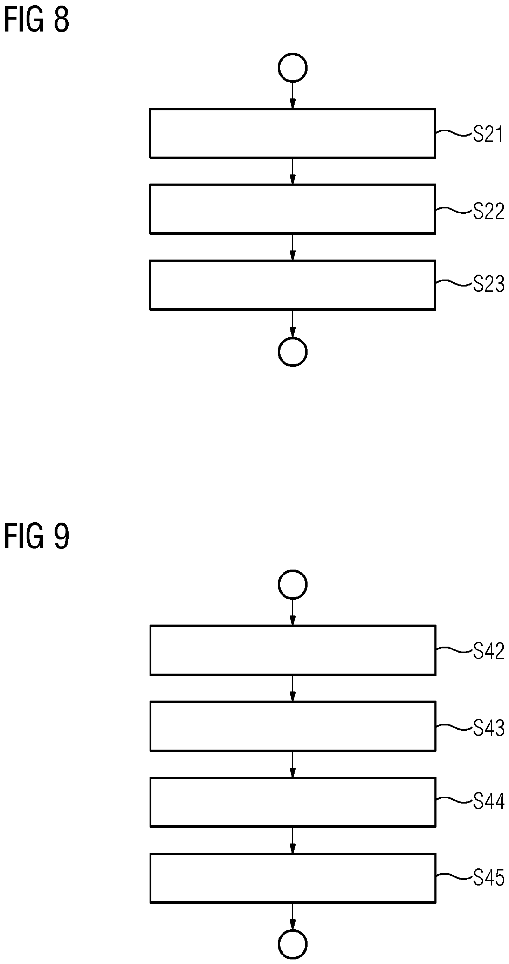

Fig. 8 zeigt schematisch einen Ablaufplan eines weiteren möglichen Teilaspektes des in Fig. 3 dargestellten Verfahrens, hier eine mögliche Störunterdrückung durch zuvor erfasste Bildinformation.

Dabei empfängt der Empfänger 70 in einem Schritt S21 ein erstes empfangenes Magnetresonanzsignal und speichert es in einem Speicher. Dabei ist es auch denkbar, dass das in Schritt S21 erfasste Signal aus einer Kalibriermessung oder einem Pre-Scan stammt, die auch mit anderen Parametern oder einer niedrigeren Auflösung erfasst werden.

In einem weiteren Schritt S22 empfängt der Empfänger 70 ein zweites empfangenes Magnetresonanzsignal und speichert dieses. Vorzugsweise handelt es sich bei dem zweiten Magnetresonanzsignal um ein Signal für eine Bilderfassung.

In einem Schritt S23 werden das erste empfangene Magnetresonanzsignal und das zweite empfangene Magnetresonanzsignal verglichen. Dies kann beispielsweise bereits in den Rohdaten erfolgen oder auch erst im Bildraum, beispielsweise nach einer Fouriertransformation. In einer bevorzugten Ausführungsform erfolgt der Vergleich auf Zeilen-Basis im k-Raum. Unterscheiden sich das erste Magnetresonanzsignal und das zweite Magnetresonanzsignal signifikant, insbesondere wenn beim Vergleich auch eine möglicherweise unterschiedliche Aufnahmesituation bereits berücksichtigt wird, so wird bei einer Abweichung, die auf externe Störer zurückzuführen ist, eine Störunterdrückungsmaßnahme ausgeführt. Störsignale können sich beispielsweise durch die Frequenz, Amplitude oder einen charakteristischen Verlauf bzw. Dauer auszeichnen.

Eine Störunterdrückungsmaßnahme kann dabei beispielsweise eine Wiederholung der Erfassung sein, was insbesondere bei einer Zeile im k-Raum zu geringeren Verzögerungen führt. Denkbar ist es auch, das Signal auf Null zu setzen, vor allem, wenn es sich um einen Bereich handeln sollte aus dem kein Bildsignal zu erwarten ist.

Fig. 9 zeigt schematisch einen Ablaufplan eines weiteren Teilaspektes eines erfindungsgemäßen Verfahrens zur Störunterdrückung, hier eine mögliche Störunterdrückung durch Analyse der erfassten Bildinformation in einem Bildraum.

In dieser möglichen Ausführungsform des erfindungsgemäßen Verfahrens wird zur Erkennung des Störsignals und zur Bestimmung der Parameter zur Entstörung das Magnetresonanzsignal im Bildraum untersucht. Dazu werden in einem Teilschritt S42 die empfangenen Magnetresonanzsignale durch die Steuerung 23 in einen Bildraum transformiert, beispielsweise durch eine Fouriertransformation. Dieser Schritt kann auch wie die nachfolgenden Schritte auf einzelne Zeilen des Rohdatenraumes erfolgen, sodass eine Erkennung und Korrektur schneller erfolgen kann.

In einem anderen Teilschritt S43 werden die Störsignale durch die Steuerung 23 von den Magnetresonanzdaten getrennt. Dies ist beispielsweise möglich, wenn durch eine Segmentierung aus einem Pre-Scan oder anderen Zusatzinformationen zu dem Patienten und Lage Bereiche im Bildraum festgelegt werden können, zu denen keine Magnetresonanzsignale von Kernspins zu erwarten sind. Dort im Bildraum auftretende Signale sind dann einer Störung zu zuordnen.

In einem weiteren Teilschritt S44 werden die Störsignale in einen Rohdatenraum zurücktransformiert, beispielsweise wieder durch eine Fouriertransformation.

In einem anderen Teilschritt S45 können dann aus den von den Nutzsignal getrennten und rücktransformierten Störsignalen im Rohdatenraum die Parameter zur Störunterdrückung bestimmt werden, beispielsweise als Phase und Amplitude zur destruktiven Interferenz im Empfänger aus den Signalen der ersten und zweiten Empfangsantennen. Denkbar ist es dabei auch, dass die Schritte des Rücktransformierens und das Bestimmen der Parameter miteinander verknüpft sein, denn Frequenz und Phase im Rohdatenraum sind mit der Position im Bildraum verknüpft.

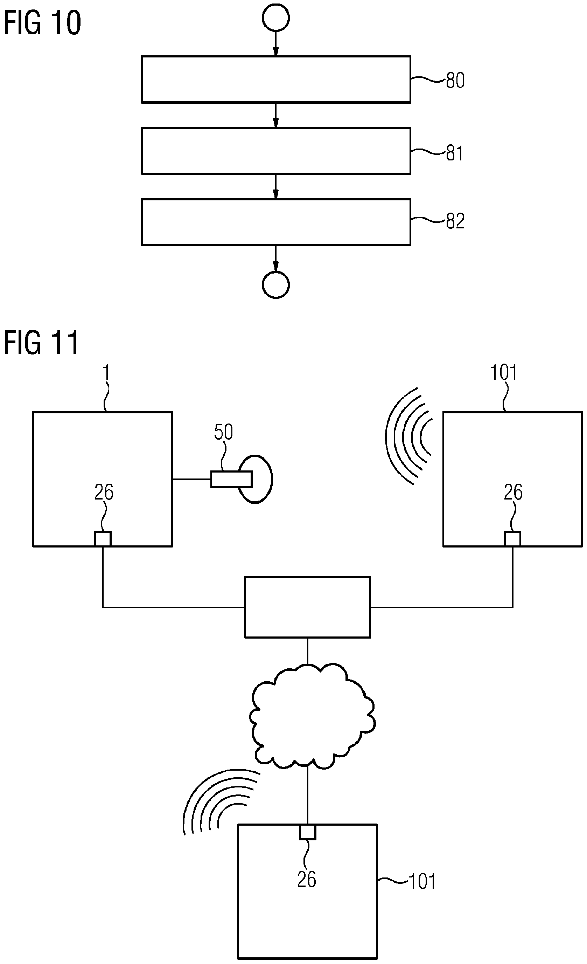

Fig. 10 zeigt schematisch einen Ablaufplan eines weiteren Teilaspektes eines erfindungsgemäßen Verfahrens zur Störunterdrückung, das Transferfunktionen zwischen ersten Empfangsantennen und zweiten Empfangsantennen 60 mit einem oder mehreren zusätzlichen Kalibrierelementen 86 ermittelt.

In einem Teilschritt S80 wird dabei eine Transferfunktion zwischen einer ersten Empfangsantenne und dem Kalibrierelement 86 ermittelt. Dazu ist es denkbar, dass über das Kalibrierelement 86 unter Koordination der Steuerung 23 von der Störunterdrückungssteuerung ein Signal ausgesendet wird, das von der ersten Empfangsantenne empfangen und ausgewertet wird. Vorzugsweise ist das Signal mittels einer Pseudozufallsfolge oder auf eine andere Art so codiert, dass eine Korrelation zwischen gesendetem und empfangenen Signal von dem Empfänger 70 leicht ermittelt werden kann.

In einem Teilschritt S82 wird auf gleiche Weise eine Transferfunktion zwischen einer ersten Empfangsantenne und dem Kalibrierelement 86 ermittelt. Dazu ist es denkbar, dass über das Kalibrierelement 86 unter Koordination der Steuerung 23 von der Störunterdrückungssteuerung ein Signal ausgesendet wird, das von der zweiten Empfangsantenne 60 empfangen und in dem Empfänger 70 ausgewertet wird.

Wegen der Umkehrbarkeit der Ausbreitung der elektromagnetischen Felder könnten aber auch von der ersten Empfangsantenne und der zweiten Empfangsantenne 62 Signale gesendet werden, die vom Kalibrierelement 86 empfangen werden.

In einem anderen Teilschritt S82 wird mindestens ein Parameter zur Empfangsentstörung in Abhängigkeit von den gemessenen Transferfunktionen derart eingestellt, dass ein Anteil eines von der zweiten Empfangsantenne 60 empfangenen Störsignals in einem von dem Empfänger 70 über die erste Empfangsantenne empfangenen Signal reduziert ist. Beispielsweise kann über die Transferfunktionen jeweils bestimmt werden, wie ein Störsignal aus der Richtung des Kalibrierelements 86 am Eingang des Empfängers 70 über die erste Empfangsantenne und die zweite Empfangsantenne 60 ankommt, insbesondere mit welcher Amplitude und Phasenverschiebung. Damit kann beispielsweise eine zusätzliche Phasenverschiebung im Empfänger 70 eingestellt werden, sodass die Signale aus erster Empfangsantenne und zweiter Empfangsantenne destruktiv im Empfänger überlagern und die Störung unterdrückt wird. Als weiterer Parameter kann die Verstärkung der Amplitude so eingestellt werden, dass für ein Störsignal von einem Punkt im Raum eine Auslöschung erfolgt. Bei mehreren ersten Empfangsantennen und zweiten Empfangsantennen 60 sind entsprechend mehr Parameter oder Parameterpaare anzupassen, was beispielsweise durch lineare Optimierungsverfahren wie LSR erfolgen kann.

In Fig. 11 ist ein Zusammenspiel aus mehreren erfindungsgemäßen Magnetresonanztomographen 1 dargestellt. Dabei empfängt die Steuereinheit 20 des ersten Magnetresonanztomographen 1 über eine Schnittstelle ein Signal von einem zweiten Magnetresonanztomographen 101. Die Steuereinheit 21 ist dabei ausgelegt, eine Bilderfassung in Abhängigkeit von einem über die Schnittstelle von dem zweiten Magnetresonanztomographen empfangenen Signal zu synchronisieren.

In der Fig. 11 sind mehrere mögliche Ausführungsformen gleichzeitig dargestellt. Die Schnittstelle kann zum einen die LAN-Schnittstelle 26 sein, über die der Magnetresonanztomograph 1 mit dem zweiten Magnetresonanztomographen 101 in Signalverbindung steht. Denkbar sind aber auch alle anderen Schnittstellen zum Informationsaustausch wie WIFI, WAN oder serielle oder parallele Punkt-zu-Punkt-Verbindungen.

Dabei ist es in einer Ausführungsform denkbar, dass der zweite Magnetresonanztomograph 101 mit dem Signal eine Nachricht über eine geplante Bilderfassung sendet. Die Nachricht kann beispielsweise angeben, dass zu einem bestimmten Zeitpunkt t auf der Frequenz f eine Anregungspuls der Dauer d von dem zweiten Magnetresonanztomograph 101 gesendet werden wird. Die Steuereinheit 20 des ersten Magnetresonanztomographen 1 synchronisiert dann die eigene Bilderfassung in Abhängigkeit von dieser Information.

Eine Möglichkeit ist, dass die Steuereinheit 20 einen eigenen Anregungspuls derart synchronisiert, dass er zur gleichen Zeit stattfindet, denn aufgrund der extrem hohen Feldstärken, die zur Anregung erforderlich sind, stören sich die Anregungspulse benachbarter Magnetresonanztomographen aufgrund der bereits durch die Konstruktion der Magnetresonanztomographen gegebenen Dämpfung nicht.

Empfindlicher gegenüber Störungen ist hingegen der Empfang von Magnetresonanzsignalen aus dem Untersuchungsvolumen bzw. dem Patienten 100. Da hier eine Dämpfung gegenüber dem Anregungspuls von über 100 dB vorliegt, kann ein Anregungspuls eines benachbarten Magnetresonanztomographen 101 den Empfang eines MR-Signals selbst bei vorhandener Abschirmung stören. Die Steuereinheit 20 des Magnetresonanztomographen 1 kann deshalb die Bilderfassung derart planen und ausführen, dass diese nicht mit dem Anregungspuls des zweiten Magnetresonanztomographen 101 zusammenfällt. Beispielsweise können eigene Anregungspulse und die davon abhängigen Auslesesequenzen so gelegt werden, dass die Empfangszeitfenster des ersten Magnetresonanztomographen 1 nicht mit den Anregungspulsen des zweiten Magnetresonanztomographen 101 zusammenfallen.

Es ist dabei auch umgekehrt möglich, dass der zweite Magnetresonanztomograph 101 eine Information über einen geplanten Empfang sendet. Die Nachricht kann beispielsweise angeben, dass zu einem bestimmten Zeitpunkt t auf der Frequenz f für die Dauer d von dem zweiten Magnetresonanztomograph 101 ein MR-Signal aufgezeichnet werden soll. Der erste Magnetresonanztomograph 1 kann dann einen eigenen Sendevorgang so einstellen, dass in dem in der Nachricht angegebenen Zeitfenster kein Sendevorgang stattfindet, zumindest nicht auf einem Frequenzband, dass die Frequenz f einschließlich einer in der Nachricht angegebenen Bandbreite umfasst.

Denkbar sind schließlich darüber hinaus kombinierte Nachrichten, in denen wechselseitig zwischen dem ersten Magnetresonanztomographen 1 und dem zweiten Magnetresonanztomographen 101 Sende- und Empfangsvorgänge abgestimmt werden, vorzugsweise derart, dass durch Verschachtelung die Bilderfassungsgeräte mit möglichst geringer Verzögerung ausgeführt werden können.

In einer anderen Ausführungsform, die in Fig. 11 dargestellt ist, ist es aber auch denkbar, dass das Signal eine Radiowelle eines Anregungspulses selbst ist und die Schnittstelle beispielsweise die Lokalspule 50 mit der Hochfrequenzeinheit 22. Vorzugsweise erfolgt dabei ein Empfang auch oder gerade in Zeiten, in denen der erste Magnetresonanztomograph 1 selbst kein MR-Signal aufzeichnet. Anhand des Anregungspulses kann die Steuereinheit 20 erfassen, dass ein zweiter Magnetresonanztomograph 101 gerade einen Anregungspuls gesendet hat und daher im Anschluss eine Erfassung eines Magnetresonanzsignals plant. Es ist dann beispielsweise denkbar, dass der erste Magnetresonanztomograph 1 dann für eine gewisse Zeit selbst keine Anregungspulse aussendet. Möglich wäre es auch, dass der erste Magnetresonanztomograph 1 den Anregungspuls des zweiten Magnetresonanztomographen 101 selbst als Trigger-Puls nutzt und nahezu synchron einen eigenen Anregungspuls aussendet, da üblicherweise zwischen Anregungspuls und Empfang des Magnetresonanzsignals Pausen ohne Empfang und damit mögliche wechselseitige Störung liegen.

Unabhängig davon, ob das Aussenden eines Anregungspulses direkt über das empfangene elektromagnetische Feld des Pulses oder über eine Nachricht über die Datenschnittstelle erkannt wird, ist es dabei auch denkbar, dass die Steuereinheit 20 die Frequenz des nächsten Anregungspulses in Abhängigkeit von dem Signal ändert. Bei der Magnetresonanztomographie werden einzelne Schichten entlang der Richtung des B0-Feldes, üblicherweise entlang der z-Achse 2, durch die ein überlagertes Gradientenfeld in z-Richtung in der Frequenz differenziert und damit unterscheidbar. Die Steuereinheit 20 kann beispielsweise die Reihenfolge der Abtastung einzelner Schichten Ändern, sodass der erste Magnetresonanztomograph 1 und der zweite Magnetresonanztomograph 101 jeweils Schichten mit unterschiedlicher Mittenfrequenz erfassen und so durch die unterschiedlichen Frequenzen ein Übersprechen bzw. eine Wechselwirkung vermieden werden. Ein zusätzlicher Freiheitsgrad, den die Steuereinheit 20 nutzen kann, ist dabei auch die Lage des Patienten 100 auf der verfahrbaren Patientenliege 30 relativ zu dem Isozentrum des Feldmagneten 10. Indem der Patient 100 um ein Stück entlang der der z-Achse verfahren wird, ändert sich durch die unterschiedliche Lage in Bezug auf das z-Gradientenfeld auch die Larmorfrequenz für eine Schicht. Der erste Magnetresonanztomograph 1 kann also durch eine Relativbewegung des Patienten entlang der z-Achse auch die gleiche Schicht im Körper des Patienten 100 mit unterschiedlichen Frequenzen erfassen, sodass eine Wechselwirkung mit dem zweiten Magnetresonanztomographen 101 vermieden werden kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den durch die Ansprüche definierten Schutzumfang der Erfindung zu verlassen.

## Patentansprüche

1. Magnetresonanztomograph, wobei der Magnetresonanztomograph (1) einen Patiententunnel (16), eine erste Empfangsantenne zum Empfang eines Magnetresonanzsignals aus einem Patienten (100) in dem Patiententunnel (16), eine zweite Empfangsantenne (60) zum Empfang eines Signals mit der Larmorfrequenz des Magnetresonanzsignals, und einen Empfänger (70) aufweist, wobei die zweite Empfangsantenne (60) außerhalb oder in der Nähe einer Öffnung des Patiententunnels (16) angeordnet ist, wobei der Empfänger (70) in Signalverbindung mit der ersten Empfangsantenne und der zweiten Empfangsantenne (60) steht und der Empfänger (70) ausgelegt ist, ein mit der zweiten Empfangsantenne (60) empfangenes Störsignal in einem von der ersten Empfangsantenne empfangenen Magnetresonanzsignal zu unterdrücken,

**dadurch gekennzeichnet, dass**

der Magnetresonanztomograph (1) einen Störunterdrückungssender (80) und eine Störunterdrückungsantenne (81) aufweist, wobei die Störunterdrückungsantenne (81) in einem Abstand zu dem Patiententunnel (16) angeordnet ist, wobei der Störunterdrückungssender (80) ausgelegt ist, ein Signal in einem Frequenzbereich eines Anregungspulses des Magnetresonanztomographen (1) über die Störunterdrückungsantenne (81) in Abhängigkeit von einem Sendeentstörparameter auszugeben, sodass in einem vorbestimmten Bereich einer Umgebung des Magnetresonanztomographen (1) durch destruktive Interferenz eine Feldstärke des Anregungspulses reduziert wird.

2.  Magnetresonanztomograph, wobei der Magnetresonanztomograph (1) einen Patiententunnel (16), eine erste Empfangsantenne zum Empfang eines Magnetresonanzsignals aus einem Patienten (100) in dem Patiententunnel (16), eine zweite Empfangsantenne (60) zum Empfang eines Signals nahe der Larmorfrequenz des Magnetresonanzsignals und einen Empfänger (70) aufweist, wobei die zweite Empfangsantenne (60) außerhalb oder in der Nähe einer Öffnung des Patiententunnels (16) angeordnet ist, wobei der Empfänger (70) in Signalverbindung mit der ersten Empfangsantenne und der zweiten Empfangsantenne (60) steht und der Empfänger (70) ausgelegt ist, ein mit der zweiten Empfangsantenne (60) außerhalb eines Frequenzbereichs des Magnetresonanzsignals empfangenes breitbandiges Störsignal in einem von der ersten Empfangsantenne empfangenen Magnetresonanzsignal zu unterdrücken,

**dadurch gekennzeichnet, dass**

der Magnetresonanztomograph (1) einen Störunterdrückungssender (80) und eine Störunterdrückungsantenne (81) aufweist, wobei die Störunterdrückungsantenne (81) in einem Abstand zu dem Patiententunnel (16) angeordnet ist, wobei der Störunterdrückungssender (80) ausgelegt ist, ein Signal in einem Frequenzbereich eines Anregungspulses des Magnetresonanztomographen (1) über die Störunterdrückungsantenne (81) in Abhängigkeit von einem Sendeentstörparameter auszugeben, sodass in einem vorbestimmten Bereich einer Umgebung des Magnetresonanztomographen (1) durch destruktive Interferenz eine Feldstärke des Anregungspulses reduziert wird.

3.  Magnetresonanztomograph nach Anspruch 1 oder 2, wobei der Magnetresonanztomograph (1) ein Kalibrierelement (86) in einer Umgebung des Magnetresonanztomographen (1) und eine Störunterdrückungssteuerung (84) aufweist, wobei die Störunterdrückungssteuerung (84) ausgelegt ist, mittels des Kalibrierelements (86) eine Feldstärke in einem Frequenzbereich eines Anregungspulses am Ort des Kalibrierelementes (86) zu erfassen und in Abhängigkeit von der erfassten Feldstärke den Sendeentstörparameter derart einzustellen, dass eine Feldstärke des Anregungspulses in einer vorbestimmten Umgebung des Kalibrierelements (86) reduziert ist.

4.  Magnetresonanztomograph nach einem der vorhergehenden Ansprüche, wobei der Störunterdrückungssender (80) ausgelegt ist, die Signale für die Störunterdrückungsantenne (81) bzw. Störunterdrückungsantennen (81) durch Phasenverschiebung und/oder Amplitudenanpassung in Abhängigkeit von einem oder mehreren Sendeentstörparametern zu erzeugen.

5.  Magnetresonanztomograph nach einem der vorhergehenden Ansprüche, wobei die Störunterdrückungsantenne (81) einen Hochfrequenzleistungsverstärker aufweist.

6.  Verfahren zum Betrieb eines Magnetresonanztomographen (1), wobei der Magnetresonanztomograph (1) einen Patiententunnel (16), eine erste Empfangsantenne zum Empfang eines Magnetresonanzsignals aus einem Patienten (100) in dem Patiententunnel (16), eine zweite Empfangsantenne (60) zum Empfang eines Signals mit der Larmorfrequenz des Magnetresonanzsignals und einen Empfänger (70) aufweist, wobei die zweite Empfangsantenne (60) außerhalb des Patiententunnels (16) oder in der Nähe einer Öffnung des Patiententunnels (16) angeordnet ist und wobei das Verfahren die Schritte aufweist:

    (S10) Empfangen eines Störsignals durch den Empfänger (70) über die zweite Empfangsantenne (60);
    (S20) Empfangen eines Magnetresonanzsignals durch den Empfänger (70) über die erste Empfangsantenne;
    (S30) Verarbeiten des Magnetresonanzsignals in Abhängigkeit von dem Störsignal durch den Empfänger (70) zu einem Empfangssignal, wobei die Abhängigkeit von einem Parameter abhängt;
    (S40) Einstellen des Parameters durch den Empfänger (70), sodass ein Anteil des Störsignals in dem Empfangssignal reduziert wird, **dadurch gekennzeichnet, dass**

der Magnetresonanztomograph (1) einen Störunterdrückungssender (80) und eine Störunterdrückungsantenne (81) aufweist,

wobei die Störunterdrückungsantenne (81) in einem Abstand zu dem Patiententunnel (16) angeordnet ist, wobei der Störunterdrückungssender (80) ein Signal in einem Frequenzbereich eines Anregungspulses des Magnetresonanztomographen (1) über die Störunterdrückungsantenne (81) in Abhängigkeit von einem Sendeentstörparameter ausgibt, sodass in einem vorbestimmten Bereich einer Umgebung des Magnetresonanztomographen (1) durch destruktive Interferenz eine Feldstärke des Anregungspulses reduziert wird.

7. Verfahren zum Betrieb eines Magnetresonanztomographen (1), wobei der Magnetresonanztomograph (1) einen Patiententunnel (16), eine erste Empfangsantenne zum Empfang eines Magnetresonanzsignals aus einem Patienten (100) in dem Patiententunnel (16), eine zweite Empfangsantenne (60) zum Empfang eines Signals nahe der Larmorfrequenz des Magnetresonanzsignals und einen Empfänger (70) aufweist, wobei die zweite Empfangsantenne (60) außerhalb des Patiententunnels (16) oder in der Nähe einer Öffnung des Patiententunnels (16) angeordnet ist und wobei das Verfahren die Schritte aufweist:

(S10) Empfangen eines Frequenzanteils des Störsignals nahe der Larmorfrequenz durch den Empfänger (70) über die zweite Empfangsantenne (60);
(S20) Empfangen eines Magnetresonanzsignals durch den Empfänger (70) über die erste Empfangsantenne;
(S30) Verarbeiten des Magnetresonanzsignals in Abhängigkeit von dem Frequenzanteil des Störsignals durch den Empfänger (70) zu einem Empfangssignal, wobei die Abhängigkeit von einem Parameter abhängt;
(S40) Einstellen des Parameters durch den Empfänger (70), sodass ein Anteil des Störsignals in dem Empfangssignal reduziert wird, **dadurch gekennzeichnet, dass**

der Magnetresonanztomograph (1) einen Störunterdrückungssender (80) und eine Störunterdrückungsantenne (81) aufweist,

wobei die Störunterdrückungsantenne (81) in einem Abstand zu dem Patiententunnel (16) angeordnet ist, wobei der Störunterdrückungssender (80) ein Signal in einem Frequenzbereich eines Anregungspulses des Magnetresonanztomographen (1) über die Störunterdrückungsantenne (81) in Abhängigkeit von einem Sendeentstörparameter ausgibt, sodass in einem vorbestimmten Bereich einer Umgebung des Magnetresonanztomographen (1) durch destruktive Interferenz eine Feldstärke des Anregungspulses reduziert wird.

8. Computerprogrammprodukt, welches direkt in einen Prozessor einer programmierbaren Steuerung (23) eines Magnetresonanztomographen nach einem der Ansprüche 1 bis 5 ladbar ist, mit Programmcode-Mitteln, um alle Schritte eines Verfahrens nach einem der Ansprüche 6 oder 7 auszuführen, wenn das Computerprogramm-produkt auf der Steuerung (23) ausgeführt wird.

9. Computerlesbares Speichermedium mit darauf gespeicherten elektronisch lesbaren Steuerinformationen, welche derart ausgestaltet sind, dass sie bei Verwendung des Speichermediums in einer Steuerung (23) eines Magnetresonanztomographen (1) nach Anspruch 1 bis 5 den Magnetresonanztomographen dazu veranlassen, das Verfahren nach einem der Ansprüche 6 oder 7 durchzuführen.

## Claims

1. MRI scanner, wherein the MRI scanner (1) has a patient tunnel (16), a first receiving antenna for receiving a magnetic resonance signal from a patient (100) in the patient tunnel (16), a second receiving antenna (60) for receiving a signal having the Larmor frequency of the magnetic resonance signal, and a receiver (70), wherein the second receiving antenna (60) is arranged outside or in the vicinity of an opening of the patient tunnel (16), wherein the receiver (70) is connected to the first receiving antenna and the second receiving antenna (60) for signalling and the receiver (70) is designed to suppress an interference signal received with the second receiving antenna (60) in a magnetic resonance signal received by the first receiving antenna, **characterised in that** the MRI scanner (1) has an interference suppression transmitter (80) and an interference suppression antenna (81), wherein the interference suppression antenna (81) is arranged at a distance from the patient tunnel (16), wherein the interference suppression transmitter (80) is designed to output a signal in a frequency range of an excitation pulse of the MRI scanner (1) via the interference suppression antenna (81) as a function of a transmit interference suppression parameter so that, in a predetermined area of an environment of the MRI scanner (1), a field strength of the excitation pulse is reduced by destructive interference.

2. MRI scanner, wherein the MRI scanner (1) has a patient tunnel (16), a first receiving antenna for receiving a magnetic resonance signal from a patient (100) in the patient tunnel (16), a second receiving antenna (60) for receiving a signal close to the Larmor frequency of the magnetic resonance signal and a receiver (70), wherein the second receiving antenna (60) is arranged outside or in the vicinity of an opening of the patient tunnel (16), wherein the receiver (70) is connected to the first receiving antenna and the second receiving antenna (60) for signalling and the receiver (70) is designed to suppress a wideband interference signal received with the second receiving antenna (60) outside a frequency range of the magnetic resonance signal in a magnetic resonance signal received by the first receiving antenna, **characterised in that** the MRI scanner (1) has an interference suppression transmitter (80) and an interference suppression antenna (81), wherein the interference suppression antenna (81) is arranged at a distance from the patient tunnel (16), wherein the interference suppression transmitter (80) is designed to output a signal in a frequency range of an excitation pulse of the MRI scanner (1) via the interference suppression antenna (81) as a function of a transmit interference suppression parameter so that, in a predetermined area of an environment of the MRI scanner (1), a field strength of the excitation pulse is reduced by destructive interference.

3. MRI scanner according to claim 1 or 2, wherein the MRI scanner (1) has a calibration element (86) in an environment of the MRI scanner (1) and an interference suppression controller (84), wherein the interference suppression controller (84) is designed to detect a field strength in a frequency range of an excitation pulse at the location of the calibration element (86) by means of the calibration element (86) and as a function of the field strength detected to set the transmit interference suppression parameter in such a way that a field strength of the excitation pulse is reduced in a predetermined environment of the calibration element (86).

4. MRI scanner according to one of the preceding claims, wherein the interference suppression transmitter (80) is designed to generate the signals for the interference suppression antenna (81) or interference suppression antennas (81) by phase displacement and/or amplitude adaptation as a function of one or more transmit interference suppression parameters.

5. MRI scanner according to one of the preceding claims, wherein the interference suppression antenna (81) has a radio frequency power amplifier.

6. Method for operation of an MRI scanner (1), wherein the MRI scanner (1) has a patient tunnel (16), a first receiving antenna for receiving a magnetic resonance signal from a patient (100) in the patient tunnel (16), a second receiving antenna (60) for receiving a signal with the Larmor frequency of the magnetic resonance signal and a receiver (70), wherein the second receiving antenna (60) is arranged outside the patient tunnel (16) or in the vicinity of an opening of the patient tunnel (16) and wherein the method has the steps:

(S10) Receipt of an interference signal by the receiver (70) via the second receiving antenna (60);
(S20) Receipt of a magnetic resonance signal by the receiver (70) via the first receiving antenna;
(S30) Processing of the magnetic resonance signal as a function of the interference signal by the receiver (70) to a receive signal, wherein the dependency is a function of a parameter;
(S40) Setting of the parameter by the receiver (70), so that a portion of the interference signal is reduced in the receive signal,
**characterised in that**
the MRI scanner (1) has an interference suppression transmitter (80) and an interference suppression antenna (81), wherein the interference suppression antenna (81) is arranged at a distance from the patient tunnel (16), wherein the interference suppression transmitter (80) outputs a signal in a frequency range of an excitation pulse of the MRI scanner (1) via the interference suppression antenna (81) as a function of a transmit interference suppression parameter so that, in a predetermined area of an environment of the MRI scanner (1), a field strength of the excitation pulse is reduced by destructive interference.

7. Method for operation of an MRI scanner (1), wherein the MRI scanner (1) has a patient tunnel (16), a first receiving antenna for receiving a magnetic resonance signal from a patient (100) in the patient tunnel (16), a second receiving antenna (60) for receiving a signal close to the Larmor frequency of the magnetic resonance signal and a receiver (70), wherein the second receiving antenna (60) is arranged outside the patient tunnel (16) or in the vicinity of an opening of the patient tunnel (16) and wherein the method has the steps:

(S10) Receipt of a frequency portion of the interference signal close to the Larmor frequency by the receiver (70) via the second receiving antenna (60);
(S20) Receipt of a magnetic resonance signal by the receiver (70) via the first receiving antenna;

(S30) Processing of the magnetic resonance signal as a function of the frequency portion of the interference signal by the receiver (70) to a receive signal, wherein the dependency is a function of a parameter;

(S40) Setting of the parameter by the receiver (70), so that a portion of the interference signal is reduced in the receive signal,

**characterised in that**

the MRI scanner (1) has an interference suppression transmitter (80) and an interference suppression antenna (81), wherein the interference suppression antenna (81) is arranged at a distance from the patient tunnel (16), wherein the interference suppression transmitter (80) outputs a signal in a frequency range of an excitation pulse of the MRI scanner (1) via the interference suppression antenna (81) as a function of a transmit interference suppression parameter so that, in a predetermined area of an environment of the MRI scanner (1), a field strength of the excitation pulse is reduced by destructive interference.

8. Computer program product, which is able to be loaded directly into a processor of a programmable controller (23) of an MRI scanner according to one of claims 1 to 5, with program code means for carrying out all steps of a method according to one of claims 6 or 7 when the computer program product is executed at the controller (23).

9. Computer-readable storage medium with electronically-readable control information stored thereon, which is designed in such a way that, when the storage medium is used in a controller (23) of an MRI scanner (1) according to claim 1 to 5, it prompts the MRI scanner to carry out the method according to one of claims 6 or 7.

**Revendications**

1. Tomodensitomètre à résonance magnétique, dans lequel le tomodensitomètre (1) à résonance magnétique comporte un tunnel (16) de patient, une première antenne de réception pour la réception d'un signal de résonnance magnétique d'un patient (100) dans le tunnel (16) de patient, une deuxième antenne (60) de réception pour la réception d'un signal ayant la fréquence de Larmor du signal de résonnance magnétique et un récepteur (70), dans lequel la deuxième antenne (60) de réception est disposée à l'extérieur ou à proximité d'une ouverture du tunnel (16) de patient,

dans lequel le récepteur (70) est en liaison de signal avec la première antenne de réception et avec la deuxième antenne (60) de réception et le récepteur (70) est conçu pour supprimer un signal parasite, reçu par la deuxième antenne (60) de réception, dans un signal de résonnance magnétique, reçu par la première antenne de réception ;

**caractérisé en ce que**

le tomodensitomètre (1) à résonnance magnétique a un émetteur (80) de suppression de parasites et une antenne (81) de suppression de parasites, dans lequel l'antenne (81) de suppression de parasites est disposée à distance du tunnel (16) de patient, dans lequel l'émetteur (80) de suppression de parasites est conçue pour donner un signal dans une plage de fréquences d'une impulsion d'excitation du tomodensitomètre (1) à résonnance magnétique par l'antenne (81) de suppression de parasites en fonction d'un paramètre de perturbation d'émission, de manière à réduire par interférence destructive, dans une partie déterminée à l'avance d'un environnement du tomodensitomètre (1) à résonnance magnétique, une intensité du champ de l'impulsion d'excitation.

2. Tomodensitomètre à résonance magnétique, dans lequel le tomodensitomètre (1) à résonance magnétique comporte un tunnel (16) de patient, une première antenne de réception pour la réception d'un signal de résonance magnétique d'un patient (100) dans le tunnel (16) de patient, une deuxième antenne (60) de réception pour la réception d'un signal ayant la fréquence de Larmor du signal de résonnance magnétique et un récepteur (70), dans lequel la deuxième antenne (60) de réception est disposée à l'extérieur ou à proximité d'une ouverture du tunnel (16) de patient,

dans lequel le récepteur (70) est en liaison de signal avec la première antenne de réception et avec la deuxième antenne (60) de réception et le récepteur (70) est conçu pour supprimer un signal parasite à bande large, reçu par la deuxième antenne (60) de réception à l'extérieur d'une plage de fréquences du signal de résonnance magnétique, dans un signal de résonance magnétique reçu par la première antenne de réception,

**caractérisé en ce que**

le tomodensitomètre (1) à résonnance magnétique a un émetteur (80) de suppression de parasites et une antenne (81) de suppression de parasites, dans lequel l'antenne (81) de suppression de parasites est disposée à distance du tunnel (16) de patient, dans lequel l'émetteur (80) de suppression de parasites est conçu pour donner un signal dans une plage de fréquences d'une impulsion d'excitation du tomodensitomètre (1) à résonnance magnétique par l'antenne (81) de suppression de parasites en fonction d'un paramètre de perturbation d'émission, de manière à réduire par interférence destructive, dans une partie déterminée à l'avance d'un environnement du tomodensitomètre (1) à résonnance magnétique, une intensité de champ de l'impulsion d'excitation.

3. Tomodensitomètre à résonnance magnétique suivant la revendication 1 ou 2, dans lequel le tomodensitomètre (1) à résonnance magnétique a un élément (86) d'étalonnage dans un environnement du tomodensitomètre (1) à résonnance magnétique et une commande (84) de suppression de parasites, dans lequel la commande (84) de suppression de parasites est conçue pour, au moyen de l'élément (86) d'étalonnage, détecter une intensité du champ dans une plage de fréquences d'une impulsion d'excitation à l'emplacement de l'élément (86) d'étalonnage et, en fonction de l'intensité du champ détecté, régler le paramètre de perturbation d'émission, de manière à réduire une intensité du champ de l'impulsion d'excitation dans un environnement déterminé à l'avance de l'élément (86) d'étalonnage.

4. Tomodensitomètre à résonnance magnétique suivant l'une des revendications précédentes, dans lequel l'émetteur (80) de suppression de parasites est conçu pour produire les signaux pour l'antenne (81) de suppression de parasites ou les antennes (81) de suppression de parasites par déphasage et/ou adaptation d'amplitude en fonction d'un ou plusieurs paramètres de perturbation d'émission.

5. Tomodensitomètre à résonnance magnétique suivant l'une des revendications précédentes, dans lequel l'antenne (81) de suppression de parasites a un amplificateur de puissance à haute fréquence.

6. Procédé pour faire fonctionner un tomodensitomètre (1) à résonnance magnétique, dans lequel le tomodensitomètre (1) à résonnance magnétique comporte un tunnel (16) de patient, une première antenne de réception pour la réception d'un signal de résonnance magnétique d'un patient (100) dans le tunnel (16) de patient, une deuxième antenne (60) de réception pour la réception d'un signal ayant la fréquence de Larmor du signal de résonnance magnétique et un récepteur (70), dans lequel la deuxième antenne (60) de réception est disposée à l'extérieur du tunnel (16) ou à proximité d'une ouverture du tunnel (16) de patient et dans lequel le procédé comporte les stades :

    (S10) réception d'un signal parasite par le récepteur (70) par l'intermédiaire de la deuxième antenne (60) de réception ;
    (S20) réception d'un signal de résonnance magnétique par le récepteur (70) par l'intermédiaire de la première antenne de réception ;
    (S30) transformation par le récepteur (70) du signal de résonnance magnétique en un signal de réception en fonction du signal parasite, dans lequel la fonction dépend d'un paramètre ;
    (S40) réglage du paramètre par le récepteur (70) de manière à réduire une proportion du signal parasite dans le signal de réception,
    **caractérisé en ce que**
    le tomodensitomètre (1) à résonnance magnétique a un émetteur (80) de suppression de parasites et une antenne (81) de suppression de parasites, dans lequel l'antenne (81) de suppression de parasites est disposée à distance du tunnel (16) de patient, dans lequel l'émetteur (80) de suppression de parasites donne un signal dans une plage de fréquences d'une impulsion d'excitation du tomodensitomètre (1) à résonnance magnétique par l'antenne (81) de suppression de parasites en fonction d'un paramètre de perturbation d'émission, de manière à réduire par interférence destructive, dans une partie déterminée à l'avance d'un environnement du tomodensitomètre (1) à résonnance magnétique, une intensité de champ de l'impulsion d'excitation.

7. Procédé pour faire fonctionner un tomodensitomètre (1) à résonnance magnétique, dans lequel le tomodensitomètre (1) à résonnance magnétique a un tunnel (16) de patient, une première antenne de réception pour la réception d'un signal de résonnance magnétique d'un patient (100) dans le tunnel (16) de patient, une deuxième antenne (60) de réception pour la réception d'un signal proche de la fréquence de Larmor du signal de résonnance magnétique et un récepteur (70), dans lequel la deuxième antenne (60) de réception est disposée à l'extérieur du tunnel (16) de patient ou à proximité d'une ouverture du tunnel (16) de patient et dans lequel le procédé a les stades :

    (S10) réception d'un signal parasite par le récepteur (70) par l'intermédiaire de la deuxième antenne (60) de réception ;
    (S20) réception d'un signal de résonnance magnétique par le récepteur (70) par l'intermédiaire de la première antenne de réception ;
    (S30) transformation par le récepteur (70) du signal de résonnance magnétique en un signal de réception en fonction du signal parasite, dans lequel la fonction dépend d'un paramètre ;
    (S40) réglage du paramètre par le récepteur (70) de manière à réduire une proportion du signal parasite dans le signal de réception,

**caractérisé en ce que**

le tomodensitomètre (1) à résonnance magnétique a un émetteur (80) de suppression de parasites et une antenne (81) de suppression de parasites, dans lequel l'antenne (81) de suppression de parasites est disposée à distance du tunnel (16) de patient, dans lequel l'émetteur (80) de suppression de parasites donne un signal dans une plage de fréquences d'une impulsion d'excitation du tomodensitomètre (1) à résonnance magnétique par l'antenne (81) de suppression de parasites en fonction d'un paramètre de perturbation d'émission, de manière à réduire par interférence destructive, dans une partie déterminée à l'avance d'un environnement du tomodensitomètre (1) à résonnance magnétique, une intensité de champ de l'impulsion d'excitation.

8. Produit de programme d'ordinateur, qui peut chargé directement dans un processeur d'une commande (23) programmable d'un tomodensitomètre à résonance magnétique suivant l'une des revendications 1 à 5, comprenant des moyens de code de programme pour exécuter tous les stades d'un procédé suivant l'une des revendications 6 ou 7, lorsque le produit de programme d'ordinateur est exécuté sur la commande (23).

9. Support de mémoire, déchiffrable par ordinateur, sur lequel est mis en mémoire des informations de commande déchiffrables électroniquement, qui sont conformées de manière à ce qu'elles font, lors de l'utilisation du support de mémoire dans une commande (23) d'un tomodensitomètre (1) à résonnance magnétique suivant la revendication 1 à 5, que le tomodensitomètre à résonnance magnétique effectue le procédé suivant l'une des revendications 6 ou 7.

FIG 1

FIG 2

FIG 3

FIG 4

22

220    221    222    223

FIG 5

16

262

261

1    260

FIG 6

FIG 7

S110

S111

S112

S120

S121

S122

S130

S140

FIG 8

FIG 9

FIG 10

FIG 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102014207843
- WO 2015150236 A
- US 2008048658 A1
- DE 102012203450 A1

### In der Beschreibung aufgeführte Nicht-Patentliteratur

- An improved shielded RF transmit coil for low-frequency NMR and MRI. **KUZMIN V V et al.** JOURNAL OF MAGNETIC RESONANCE. ACADEMIC PRESS, 14. Mai 2015, vol. 256, 70-76
- EXAMPLES OF THE DESIGN OF SCREENED AND SHIELDED RF RECEIVER COILS. **BURL M et al.** MAGNETIC RESONANCE IN MEDICINE. WILEY-LISS, 01. August 1996, vol. 36, 326-330